# EUROPÄISCHE PATENTANMELDUNG

(11) **EP 2 072 512 A1**
(43) Veröffentlichungstag der Anmeldung: **24.06.2009**
(21) Anmeldenummer: 07024727.5
(22) Anmeldetag: 20.12.2007
(51) Int. Cl.: C07D 413/04, C07D 413/14, C07D 498/04, A01N 43/80, A01N 43/90, A01P 13/00

(54) **Herbizide Verbindungen auf Basis von N-Azinyl-N'-pyridylsulfonyl-harnstoffen**

(71) Anmelder: Bayer CropScience AG, 40789 Monheim (DE)
(72) Erfinder: Müller, Klaus-Helmut, 40593 Düsseldorf (DE); Gesing, R. F. Ernst, 40699 Erkrath-Hochdahl (DE); Waldraff, Christian, 61118 Bad Vilbel (DE); Kehne, Heinz, 65719 Hofheim (DE); Feucht, Dieter, 65760 Eschborn (DE); Hills, Martin, 65510 Idstein (DE); Rosinger, Chris, 65719 Hofheim (DE); Dittgen, Jan, 60316 Frankfurt am Main (DE)

(57) **Zusammenfassung**

Beschrieben werden Verbindungen der allgemeinen Formel (I) 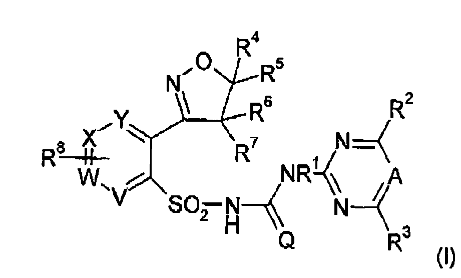 in welcher die jeweiligen Substituenten die in der Beschreibung angegebenen Bedeutungen aufweisen. Die Verbindungen der allgemeinen Formel (I) können als Herbizide und Pflanzenwachstumsregulatoren verwendet werden.

## Beschreibung

Die vorliegende Erfindung betrifft N-Azinyl-N'-pyridylsulfonyl-hamstoffe. Weiterer Gegenstand der vorliegenden Erfindung sind Mischungen der zuvor genannten Harnstoff-Derivate mit anderen Herbiziden und/oder Safenem. Darüber hinaus betrifft die vorliegende Erfindung Verfahren zur Herstellung der zuvor genannten Harnstoff-Derivate sowie die Verwendung dieser Verbindungen als Herbizide und Pflanzenwachstumsregulatoren allein und in Mischung mit Safenern und/oder in Mischung mit anderen Herbiziden, insbesondere deren Verwendung zur Pflanzenbekämpfung in speziellen Pflanzenkulturen oder als Pflanzenschutzregulatoren.

Es ist bereits bekannt, dass bestimmte N-Azinyl-N'-arylsulfonylhamstoffe mit einfachen offenkettigen Hydroxamsäureester-Gruppen im Arylteil, wie z.B. N-(4,6-Dimethylpyrimidin-2-yl)-N'-(2-methoxyaminocarbonyl-phenylsulfonyl)-harnstoff und der entsprechende N'-(2-n-Octyloxyaminocarbonylphenylsulfonyl)-hamstoff herbizide Eigenschaften aufweisen (vgl. DE 3 516 435 A, EP 0173 958 A, US 4,704,158).

Weiterhin sind auch bestimmte herbizid wirksame N-Azinyl-N'-hetarylsulfonyl-hamstoffe bekannt, welche im Hetarylteil durch O,N-dialkylierte, ebenfalls offenkettige Hydroxamsäure-Gruppen substituiert sind (vgl. EP 0 301 784 A); entsprechende cyclische Hydroxamsäurederivate sind dagegen bisher nicht beschrieben worden.

Die gemäß den oben genannten Schriften bereits bekannten Wirkstoffe weisen bei ihrer Anwendung Nachteile auf, sei es,
(a) dass sie keine oder aber eine nur unzureichende herbizide Wirkung gegen Schadpflanzen besitzen,
(b) dass nur ein zu geringes Spektrum an Schadpflanzen, bekämpft werden kann, oder
(c) dass sie eine zu geringe Selektivität in Nutzpflanzenkulturen besitzen.

Es ist deshalb wünschenswert, alternative chemische Wirkstoffe auf Basis von entsprechenden Harnstoff-Derivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Damit ergibt sich im Allgemeinen als Aufgabe der vorliegenden Erfindung, entsprechende alternative Harnstoff-Derivate bereitzustellen, welche als Herbizide oder Pflanzenwachstumsregulatoren, insbesondere mit einer zufriedenstellenden herbiziden Wirkung gegen Schadpflanzen, mit einem breiten Spektrum gegenüber Schadpflanzen und/oder mit einer hohen Selektivität in Nutzpflanzenkulturen, eingesetzt werden können. Diese Harnstoff-Derivate sollten dabei vorzugsweise ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen, als die aus dem Stand der Technik bekannten Harnstoff-Derivate zeigen.

Es wurden nun neue N-Azinyl-N'-pyridylsulfonyl-hamstoffe der allgemeinen Formel (I), 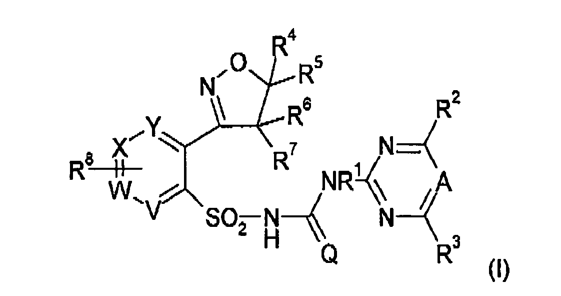 in welcher
- V, W, X und Y: so gewählt werden, dass eine dieser Indizes für Stickstoff steht und die übrigen Indizes für Kohlenstoffatome stehen, welche unsubstituiert oder aber mit dem dargestellten Rest R⁸ substituiert sein können;
- A: ausgewählt ist aus der Gruppe, bestehend aus Stickstoff und CR⁹;
wobei
R⁹ ausgewählt ist aus der Gruppe, bestehend aus Wasserstoff, Alkyl, Halogen und Haloalkyl;
- R¹: ausgewählt ist aus der Gruppe, bestehend aus Wasserstoff und einem gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl;
- R²: ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino;
- R³: ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino,
- R⁴ bis R⁷,: jeweils unabhängig voneinander, ausgewählt werden aus der Gruppe, bestehend aus Wasserstoff, Halogen, Cyano, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können, oder R⁴ und R⁶ bzw. R⁵ und R⁷ eine gegebenenfalls durch Sauerstoff oder Schwefel unterbrochene Alkyliden-Gruppe darstellen,
- R⁸: ausgewählt ist aus der Gruppe, bestehend aus Wasserstoff, Halogen, Cyano, Thiocyanato, Nitro, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl, oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können.
- Q: ausgewählt ist aus der Gruppe, bestehend aus Sauerstoff oder Schwefel, insbesondere Sauerstoff ist,
sowie Salze von Verbindungen der Formel (I).

Von einer ersten Ausführungsform der vorliegenden Erfindung werden Verbindungen der allgemeinen Formel (I) umfasst, in welchen
- A: bevorzugt ausgewählt ist aus der Gruppe, bestehend aus Stickstoff und CH.

Von einer zweiten Ausführungsform der vorliegenden Erfindungen werden Verbindungen der allgemeinen Formel (I) umfasst, in welchen
- R¹: bevorzugt ausgewählt ist aus der Gruppe, bestehend aus Wasserstoff, Alkyl, Alkoxy, Alkoxyalkyl, Alkenyl und Alkinyl, wobei die Reste unsubstituiert sein können oder ein oder mehrere Halogenatome tragen können,
- R¹: besonders bevorzugt ausgewählt ist aus der Gruppe, bestehend aus Wasserstoff, Methyl, Ethyl, Methoxy, Methoxymethyl und Ethoxy,
- R¹: insbesondere bevorzugt ausgewählt ist aus der Gruppe, bestehend aus Wasserstoff und Methyl,
und
- R¹: speziell bevorzugt Wasserstoff ist.

Von einer dritten Ausführungsform der vorliegenden Erfindung werden Verbindungen der allgemeinen Formel (I) umfasst, in welchen
- R²: bevorzugt ausgewählt ist aus der Gruppe, bestehend aus Wasserstoff, Halogen, Alkyl, Alkoxy, Alkylthio, Alkylamino und Dialkylamino, wobei die Reste unsubstituiert sein können oder ein oder mehrere Halogenatome tragen können,
- R²: besonders bevorzugt ausgewählt ist aus der Gruppe, bestehend aus Wasserstoff, Chlor, Methyl, Ethyl, Trifluoromethyl, Methoxy, Ethoxy, Trifluorethoxy, Difluoromethoxy, Methylthio, Methylamino und Dimethylamino,
und
- R²: speziell bevorzugt ausgewählt ist aus der Gruppe, bestehend aus Wasserstoff, Chlor, Methyl, Methoxy, Methylthio und Dimethylamino.

Von einer vierten Ausführungsform der vorliegenden Erfindung werden Verbindungen der allgemeinen Formel (I) umfasst, in welchen
- R³: bevorzugt ausgewählt ist aus der Gruppe, bestehend aus Wasserstoff, Halogen, Alkyl, Alkoxy, Alkylthio, Alkylamino und Dialkylamino, wobei die Reste unsubstituiert sein können oder ein oder mehrere Halogenatome tragen können,
- R³: besonders bevorzugt ausgewählt ist aus der Gruppe, bestehend aus Wasserstoff, Chlor, Methyl, Ethyl, Trifluoromethyl, Methoxy, Ethoxy, Trifluorethoxy, Difluoromethoxy, Methylthio, Methylamino oder Dimethylamino
und
- R³: speziell bevorzugt ausgewählt ist aus der Gruppe, bestehend aus Methyl, Methoxy und Trifluorethoxy.

Von einer fünften Ausführungsform der vorliegenden Erfindung werden Verbindungen der allgemeinen Formel (I) umfasst, in welchen
- R⁴ bis R⁷,: jeweils unabhängig voneinander, bevorzugt ausgewählt sind aus der Gruppe, bestehend aus Wasserstoff, Halogen, Cyano, ,Alkyl, Alkoxy, Alkylthio, Alkylsulfinyl, Alkylsulfonyl, Alkylamino, Dialkylamino, Alkylcarbonyl, Alkoxycarbony, Alkylaminocarbonyl;oder Dialkylamino, wobei die Reste unsubstituiert sein können oder ein oder mehrere Halogenatome tragen können,
- R⁴ bis R⁷,: jeweils unabhängig voneinander, besonders bevorzugt ausgewählt sind aus der Gruppe, bestehend aus Wasserstoff, Fluor, Chlor, Cyano, Methyl, Ethyl, Propyl, Isopropyl, Cyclopropyl, n-, i- s- oder tert. Butyl,Methylthio, Methylsulfinyl, Methylsulfonyl, Methoxycarbonyl und Ethoxy-carbonyl, wobei die Reste unsubstituiert sein können oder ein oder mehrere Halogenatome tragen können, und R⁴ und R⁶ bzw. R⁵ und R⁷ eine CH₂-CH₂-CH₂-Gruppe, eine CH₂-CH₂-CH₂-CH₂-Gruppe, eine CH₂-O-CH₂-Gruppe, CH₂-S-CH₂-Gruppe, eine CH₂-O-CH₂-CH₂-Gruppe, eine CH₂-CH₂-O-CH₂-Gruppe oder CH₂-CH₂-O-CH₂-CH₂-Gruppe darstellen,
und
- R⁴ bis R⁷,: jeweils unabhängig voneinander, speziell bevorzugt ausgewählt werden aus der Gruppe, bestehend aus Wasserstoff, Methyl, Ethyl, Propyl und Isopropyl, oder R⁴ und R⁶ bzw. R⁵ und R⁷ eine CH₂-CH₂-CH₂-Gruppe, eine CH₂-CH₂-CH₂-CH₂-Gruppe, eine CH₂-O-CH₂-Gruppe, CH₂-S-CH₂-Gruppe, eine CH₂-O-CH₂-CH₂-Gruppe, eine CH₂-CH₂-O-CH₂-Gruppe oder CH₂-CH₂-O-CH₂-CH₂-Gruppe darstellen.

Von einer sechsten Ausführungsform der vorliegenden Erfindung werden Verbindungen der allgemeinen Formel (I) umfasst, in welchen
- R⁸: bevorzugt ausgewählt ist aus der Gruppe, bestehend aus Wasserstoff, Halogen, Cyano, Thiocyanato, Nitro, Alkyl, Alkoxy, Alkylthio, Alkylsulfinyl, Alkylsulfonyl, Alkylamino, Dialkylamin, Alkylcarbonyl, Alkoxycarbonyl, Alkylaminocarbonyl und Dialkylaminocarbonyl, wobei die Reste unsubstituiert sein können oder ein oder mehrere Halogenatome tragen können,
und
- R⁸: besonders bevorzugt ausgewählt ist aus der Gruppe, bestehend aus Wasserstoff, Fluor, Chlor, Brom, Iod, Cyano, Methyl, Methoxy, Ethoxy, Methylthio, Ethylthio, Methylsulfinyl, Ethylsulfinyl, Methylsulfonyl, Ethylsulfonyl, Methylamino und Dimethylamino, wobei die Reste unsubstituiert sein können oder ein oder mehrere Halogenatome tragen können,
- R⁸: speziell bevorzugt Wasserstoff ist

Im Rahmen dieser Ausführungsformen der vorliegenden Erfindung ist es möglich, die einzelnen allgemeinen, bevorzugten und besonders bevorzugten Bedeutungen für die Substituenten R¹ bis R⁸, Q und A beliebig miteinander zu kombinieren. Das heißt, dass Verbindungen der allgemeinen Formel (I) von der vorliegenden Erfindung umfasst sind, in welchen beispielswiese der Substituent R¹ eine bevorzugte Bedeutung aufweist und die Substituenten R² bis R⁸ die allgemeine Bedeutung aufweisen oder aber beispielsweise der Substituent R² eine bevorzugte Bedeutung aufweist, der Substituent R³ eine besonders bevorzugte Bedeutung, der und die übrigen Substituenten die allgemeine Bedeutung aufweisen. Diese einzelnen Kombinationen werden aus Übersichtsgründen nicht expressis verbis genannt, gelten aber im Rahmen der vorliegenden Erfindung umfasst.

Der mit dem Dihydrodioxazin-Ring unmittelbar verbundene Heterocyclus der Verbindungen der allgemeinen Formel (I) 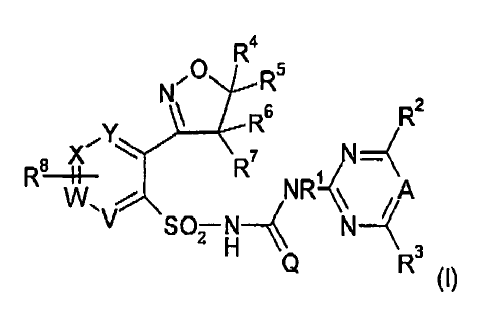 weist ein Stickstoffatom auf, d.h. ein Rest, ausgewählt aus der Gruppe, bestehend aus V, W, X und Y, entspricht einem Stickstoffatom. Die übrigen drei Reste weisen die Bedeutung eines Kohlenstoffatoms auf, wobei die Kohlenstoffatome gegebenenfalls mit dem Rest R⁸ substituiert sein können.

In einer besonderen Ausgestaltung der vorliegenden Erfindung weist die Verbindung der allgemeinen Formel (I) daher die folgende Struktur (Ia) auf, in welcher V die Bedeutung eines Stickstoffatoms hat 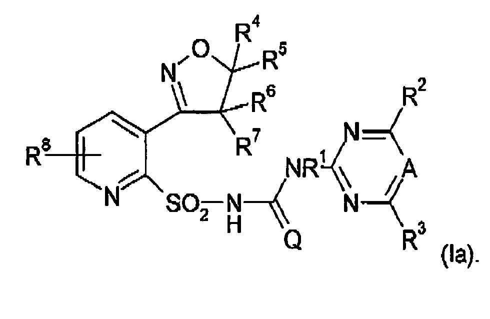 In einer noch weiteren Ausgestaltung der vorliegenden Erfindung weist die Verbindung der allgemeinen Formel (I) daher die folgende Struktur (Ib) auf, in welcher W die Bedeutung eines Stickstoffatoms hat: 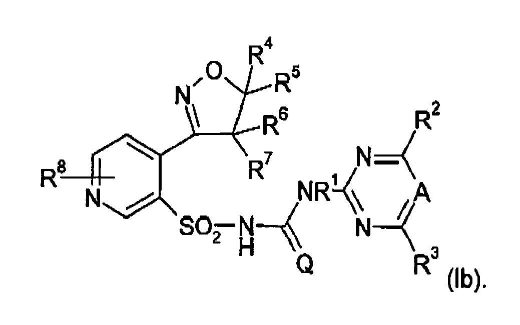 In einer noch weiteren Ausgestaltung der vorliegenden Erfindung weist die Verbindung der allgemeinen Formel (I) daher die folgende Struktur (Ic) auf, in welcher X die Bedeutung eines Stickstoffatoms hat: 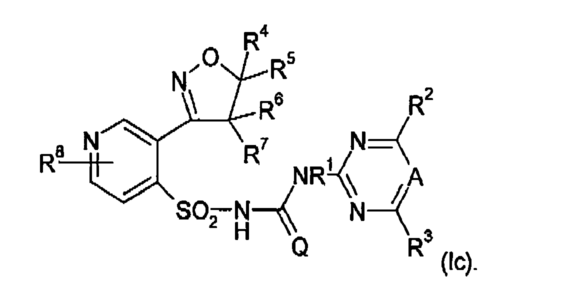 In einer noch weiteren Ausgestaltung der vorliegenden Erfindung weist die Verbindung der allgemeinen Formel (I) daher die folgende Struktur (Id) auf, in welcher Y die Bedeutung eines Stickstoffatoms hat: 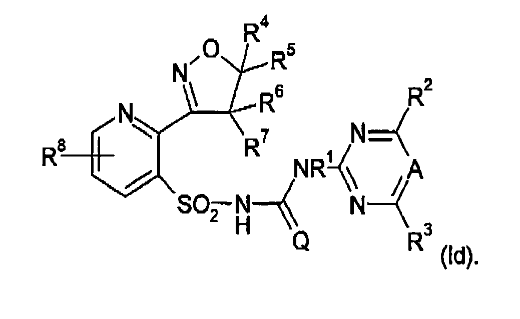 In jeder dieser vier Ausgestaltungen können die einzelnen Reste R¹ bis R⁸, A und Q die vorstehend definierten allgemeinen, bevorzugten und besonders bevorzugten Bedeutungen aufweisen.

Von diesen vier Ausgestaltungen sind erfindungsgemäß diejenigen Verbindungen besonders bevorzugt, in welchen V, W und Y die Bedeutung Stickstoff aufweisen (Verbindungen der allgemeinen Formel (Ia), (Ib), (Id)).

Insbesondere bevorzugt sind diejenigen Verbindungen der Formel (I), in welchen V und Y die Bedeutung Stickstoff aufweisen (Verbindungen der allgemeinen Formel (Ia), (Id))

Insbesondere ganz bevorzugt sind diejenigen Verbindungen der Formel (I), in welchen V die Bedeutung Stickstoff aufweist (Verbindungen der allgemeinen Formel (Ia),

In den Verbindungen der allgemeinen Formel (I) weisen die Substituenten und Reste R¹ bis R⁸, Q und A die vorstehend allgemeinen, bevorzugten, besonders bevorzugten insbesondere bevorzugten und insbesondere ganz bevorzugten Bedeutungen auf.

Gegenstand der vorliegenden Erfindung sind vorzugsweise auch die Lithium-, Natrium-, Kalium-, Magnesium-, Calcium-, Ammonium-, C₁-C₄-Alkyl-ammonium, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium, C₅- oder C₆-Cycloalkyl-ammonium-, Di-(C₁-C₂-alkyl)-benzyl-ammonium und Tri-(C₁-C₂-alkyl)-benzyl-ammonium-Salze von Verbindungen der Formel (I), in welcher R¹ bis R⁸, A und Q die oben allgemeinen, bevorzugten, besonders bevorzugten und insbesondere bevorzugten Bedeutungen aufweisen und die nach allgemein üblichen Verfahren hergestellt werden können.

Die Verbindungen der allgemeinen Formel (I) können darüber hinaus gegebenenfalls durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z. B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z. B. Sulfonsäuren oder Carbonsäuren, der Wasserstoff durch ein im agrochemischen Bereich geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze mit Kationen der Formel [NRR'R"R"']⁺, worin R bis R"' jeweils unabhängig einen organischen Rest, insbesondere Alkyl, Aryl, Aralkyl oder Alkylaryl darstellen.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, Alkylsulfinyl, Alkylsulfonyl, Haloalkylsulfinyl und Haloalkylsulfonyl, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, Propyle wie n- oder i-Propyl, Butyle wie n-, iso- oder tert.-Butyl, Pentyle wie n-Pentyl, iso-Pentyl oder neo-Pentyl, Hexyle wie n-Hexyl, i-Hexyl, 3-Methylpentyl, 2,2-Dimethylbutyl oder 2,3-Dimethylbutyl, Heptyle, wie n-Heptyl, 1-Methylhexy oder 1,4-Dimethylpentyl, Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl. Der Begriff "Halo" wird erfindungsgemäß synonym zu "Halogen" verwendet.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen (sofern nicht andersweitig definiert), sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Mit der Definition "mit einem oder mehreren Resten substituiert ist" sind, wenn nicht anders definiert, ein oder mehrere gleiche oder verschiedene Reste gemeint.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bei Resten mit C-Atomen sind solche mit 1 bis 6 C-Atomen, vorzugsweise 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano.

Wenn ein Arylrest substituiert ist, so kann es sich vorzugsweise um Phenyl, das ein oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)- Halogenalkyl, (C₁-C₄)-Halogenalkoxy, Cyano und Nitro substituiert ist, z.B. o-, m- und p-Tolyl, Dimethylphenyle, 2-, 3- und 4-Chlorphenyl, 2-, 3- und 4-Trifluormethyl und 2-, 3- und 4-Trichlormethyl-phenyl, 2,4-, 3,5-, 2,5- und 2,3-Dichlorphenyl, o-, mund p-Methoxyphenyl.

### Herstellung der erfindungsgemäßen Verbindungen der allgemeinen Formel (I)

Weiterer Gegenstand der vorliegenden Erfindung sind Verfahren zur Herstellung entsprechender Verbindungen der allgemeinen Formel (I) und/oder deren Salze.

In einer ersten Ausführungsform der vorliegenden Erfindung erfolgt die Herstellung der Verbindungen der allgemeinen Formel (I) dadurch, dass man (4,5-Dihydroisoxazol-3-yl)pyridin-sulfonamide der allgemeinen Formel (II) 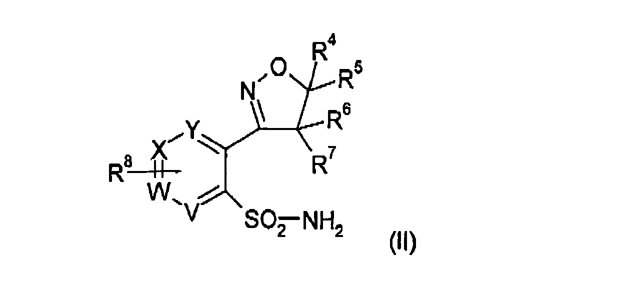 mit einem heterocyclischen (Thio)-Carbamat der allgemeinen Formel (III) 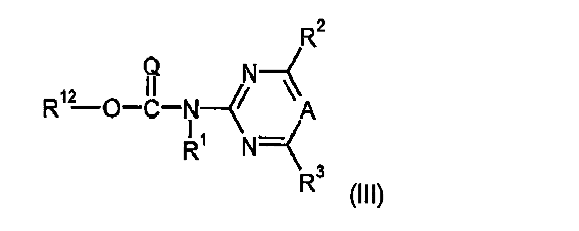 umsetzt, worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet und worin V, W, X, Y, R¹ bis R⁸, Q und A die vorstehende Bedeutung aufweisen.

Die Verbindungen der allgemeinen Formel (II) können dabei durch Umsetzung der Verbindungen der allgemeinen Formel (X) mit einem Chlorierungsmittel wie Chlorgas und Ammoniaklösung gemäß nachfolgendem Reaktionsschema erhalten werden:

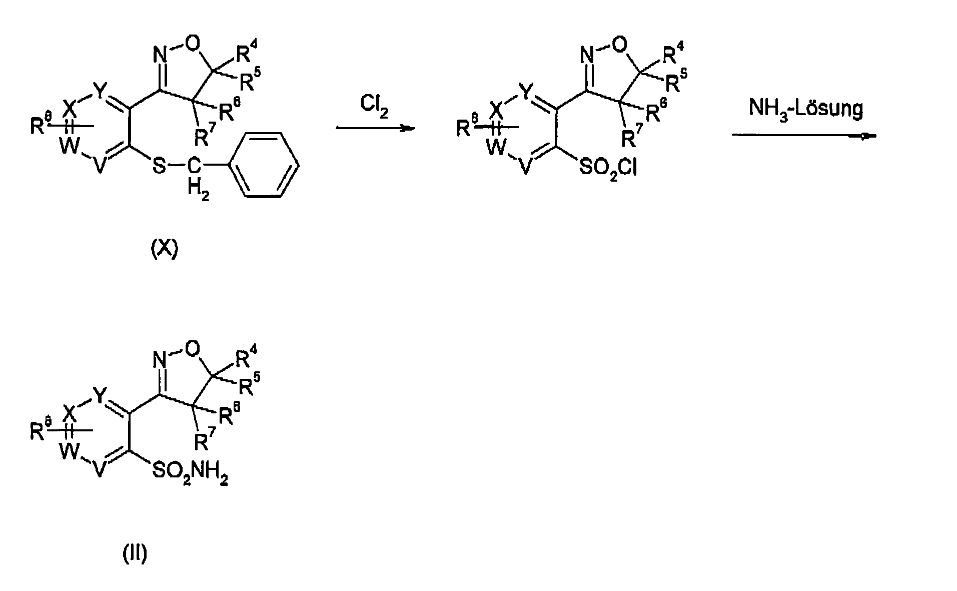

Die Verbindungen der allgemeinen Formel (X) können wiederum durch Umsetzung von Verbindungen der allgemeinen Formel (XI) mit Benzylmercaptan gemäß nachfolgendem Reaktionsschema ausgehend von den Verbindungen der allgemeinen Formel (XI) erhalten werden: 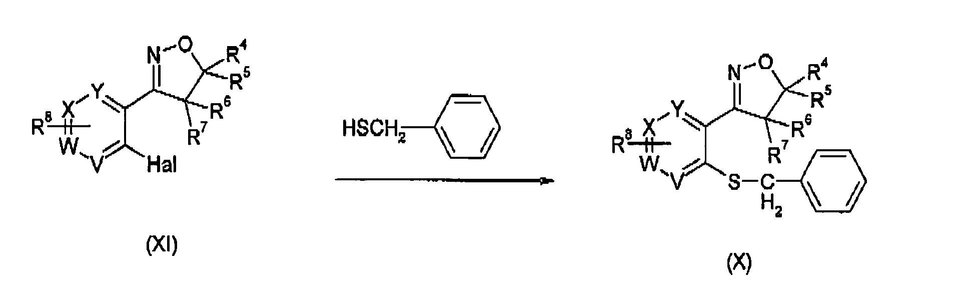 Die Verbindungen der allgemeinen Formel (XI) können wiederum durch Ringgschluss und Aufbau des Heterocyclus ausgehend von Verbindungen der allgemeinen Formel (XII) gemäß nachfolgendem Reaktionsschema erhalten werden: 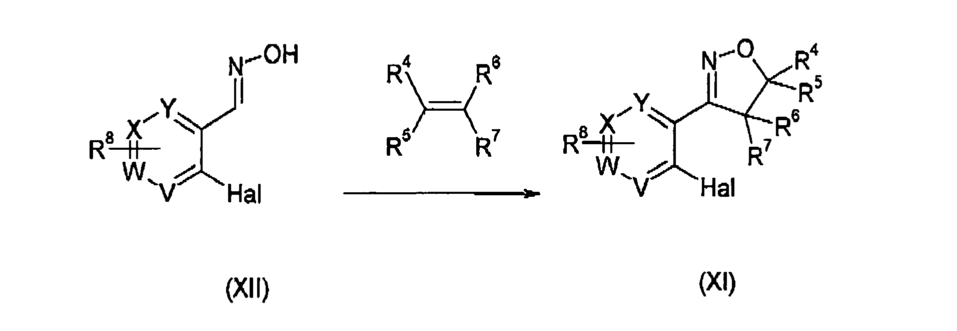 2-Chloronicotinaldehyd-oxim und substituierte 2-Chloronicotinaldehyd-oxime sind beispielsweise aus dem Stand der Technik bekannt und können nach dem Fachmann bekannten Methoden hergestellt werden (vgl. WO 2003/090539, Helvetica Chimica Acta 59, 211-21 (1976), EP 0 012 158).

In einer zweiten Ausführungsform der vorliegenden Erfindung erfolgt die Herstellung der Verbindungen der allgemeinen Formel (I) dadurch, dass man (4,5-Dihydroisoxazol-3-yl)pyridin-sulfonsäureiso(thio)cyanate der allgemeinen Formel (IV) 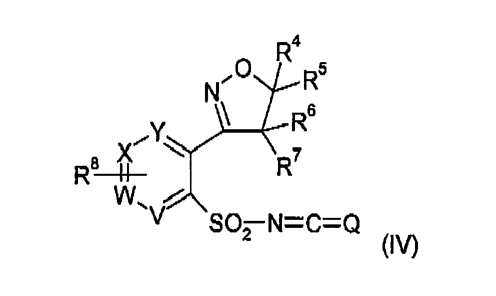 mit einem Aminoheterocyclus der allgemeinen Formel (V) 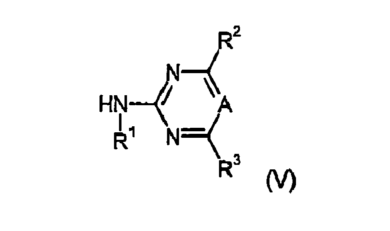 umsetzt, worin V, W, X, Y, R¹ bis R⁸, Q und A die vorstehende Bedeutung aufweisen.

In einer dritten Ausführungsform der vorliegenden Erfindung erfolgt die Herstellung der Verbindungen der allgemeinen Formel (I) dadurch, dass man Sulfonyl(thio)carbamate der allgemeinen Formel (VI) 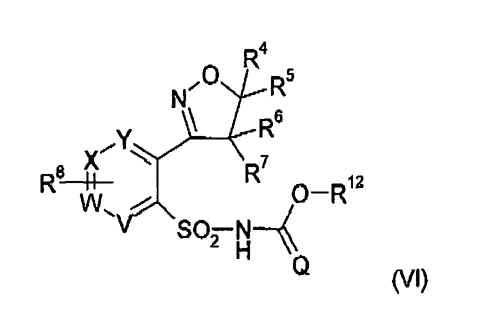 worin R¹² ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet, mit einem Aminoheterocyclus der allgemeinen Formel (V) 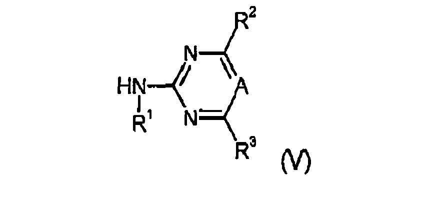 umsetzt, worin V, W, X, Y, R¹ bis R⁸, Q und A die vorstehende Bedeutung aufweisen.

In einer vierten Ausführungsform der vorliegenden Erfindung erfolgt die Herstellung der Verbindungen der allgemeinen Formel (I) dadurch, dass man (4,5-Dihydroisoxazol-3-yl)pyridin-sulfonamide der allgemeinen Formel (II) 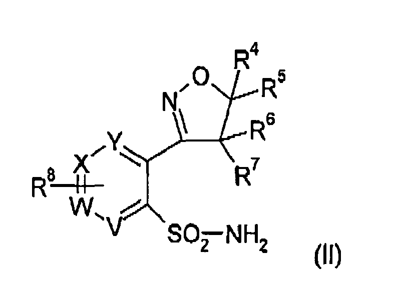 mit einem Iso(thio)cyanat der allgemeinen Formel (VII) 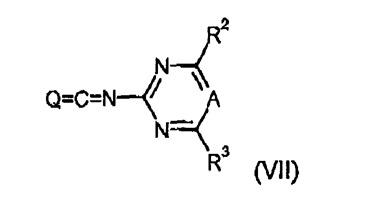 gegebenenfalls in Gegenwart eines Reaktionshilfsmittels umsetzt, worin R¹ Wasserstoff bedeutet und R² bis R⁸, V, W, X, Y, Q und A die vorstehende Bedeutung aufweisen.

In einer fünften Ausführungsform der vorliegenden Erfindung erfolgt die Herstellung der Verbindungen der allgemeinen Formel (I) dadurch, dass man einen Aminoheterocyclus der allgemeinen Formel (V) 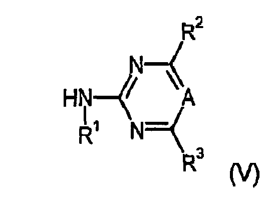 zunächst basenkatalysiert mit einem Kohlensäureester, z.B. Diphenylcarbonat, umsetzt und das gebildete Intermediat der allgemeinen Formel (III)

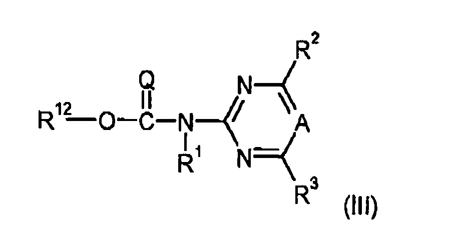

in einer Eintopfreaktion mit einem (4,5-Dihydroisoxazol-3-yl)pyridin-sulfonamid der allgemeinen Formel (II) 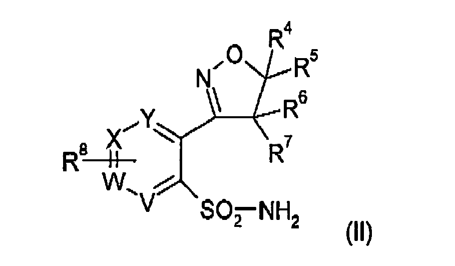 umsetzt (vgl. JP1989221386), worin V, W, X, Y, R¹ bis R⁸, Q und A die vorstehende Bedeutung aufweisen.

In einer sechsten Ausführungsform der vorliegenden Erfindung erfolgt die Herstellung der Verbindungen der allgemeinen Formel (I) dadurch, dass man (4,5-Dihydroisxazol-3-yl)pyridin-sulfonsäurehalogenide der allgemeinen Formel (VIII) 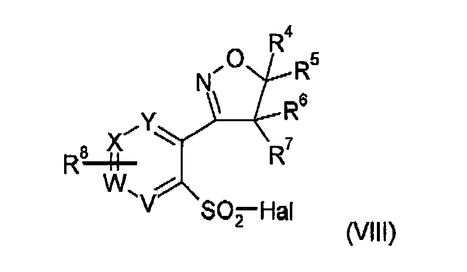 wobei Hal ein Halogenatom, vorzugsweise Chlor ist, mit einem (Thio)-Cyanat, beispielsweise einem Metall(thio)-cyanat, insbesondere einem Alkalimetall(thio)-cyanat, wie Natrium(thio)cyanat, zu einem Iso(thio)cyanat der Formel (IV) 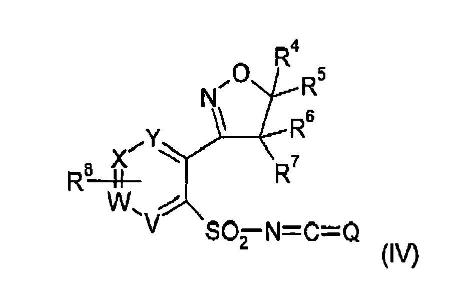 oder einem solvatisierten (stabilisierten) Derivat davon umsetzt, und anschließend mit einem Aminoheterocyclus der allgemeinen Formel (V) 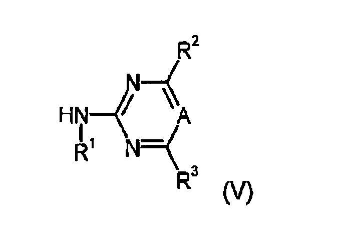 umsetzt, worin V, W, X, Y, R¹ bis R⁸, Q und A die vorstehende Bedeutung aufweisen.

In einer siebten Ausführungsform der vorliegenden Erfindung erfolgt die Herstellung der Verbindungen der allgemeinen Formel (I) mit Q = Sauerstoff dadurch, dass man (4,5-Dihydroisoxazol-3-yl)pyridin-sulfonsäureamide der allgemeinen Formel (II) 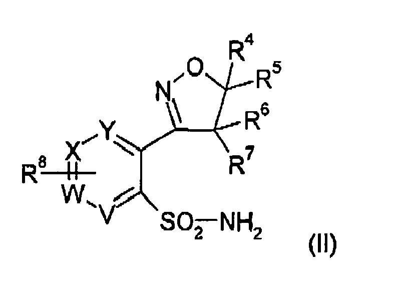 mit einem heterocyclischen Bis-carbamat der allgemeinen Formel (IX), 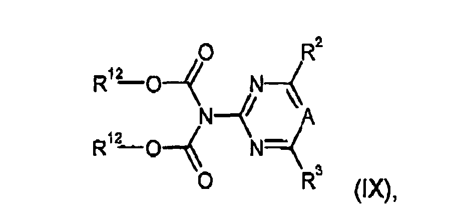 worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet, in Gegenwart eines basischen Reaktionshiffsmittels umsetzt, worin V, W, X, Y, R¹ bis R⁸ und A die vorstehende Bedeutung aufweisen.

In einer achten Ausführungsform der vorliegenden Erfindung erfolgt die Herstellung der Verbindungen der allgemeinen Formel (I) dadurch, dass man (4,5-Dihydroisoxazol-3-yl)pyridin-sulfonsäureamide der allgemeinen Formel (II) 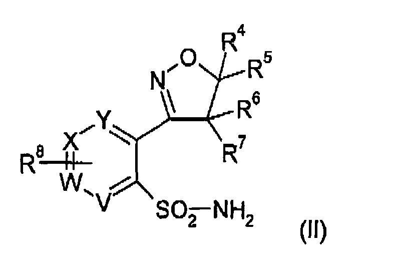 zunächst basenkatalysiert mit einem Kohlensäureester, z.B. Diphenylcarbonat, umsetzt und das gebildete Intermediat der allgemeinen Formel (VI) 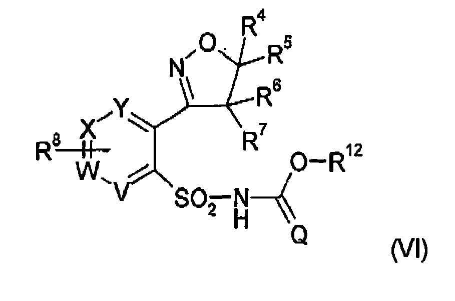 in einer Eintopfreaktion mit einem Aminoheterocyclus der allgemeinen Formel (V) 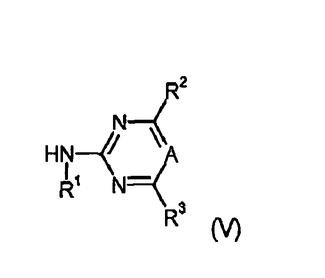 umsetzt, worin V, W, X, Y, R¹ bis R⁸, Q und A die vorstehende Bedeutung aufweisen.

Alle dieser Verfahren führen zu erfindungsgemäßen Verbindungen der allgemeinen Formel (I).

In den jeweils vorstehend genannten Verfahrensvarianten werden jeweils inerte Lösemittel verwendet. Unter inerte Lösemittel werden im Sinne der vorliegenden Erfindung Lösemittel verstanden, die unter den jeweiligen Reaktionsbedingungen inert sind, d.h. insbesondere nicht mit den Edukten reagieren, jedoch nicht unter beliebigen Reaktionsbedingungen inert sein müssen.

Beispiele organischer Lösemittel, welche im Rahmen der vorliegenden Erfindung verwendet werden können, sind aromatische oder aliphatische Lösemittel, wie Benzol, Toluol, Xylol, Mesitylen, Hexan, Heptan, Octan, Cyclohexan,; aliphatische und aromatische Halogenkohlenwasserstoffe, wie Methylenchlorid, Dichlorethan, Chloroform, Kohlenstofftetrachlorid, Chlorbenzol, Dichlorbenzol, Ether, wie Diethylether, Dibutylether, Diisobutylether, Methyl-tert-butylether, Isopropylethylether, Diisopropylether, Tetrahydrofuran, und Dioxan; weiter auch Dimethylsulfoxid, und Säureamidderivate, wie N,N-Dimethylformamid, N,N-Dimethylacetamid und N-Methyl-2-pyrrolidon, sowie auch Carbonsäureester, wie Ethylacetat, oder aber auch Diglyme, Dimethylglycol; Nitrile wie Acetonitril, Propionitril oder Butyronitril sowie Ketone wie Aceton, Methylethyl-keton oder Cyclohexanon. Besonders bevorzugt sind Toluol, Xylol, Dichlorbenzol, Chlorbenzol, Acetonitril, Aceton, Butyronitril oder Ethylacetat. Allerdings ist die vorliegende Erfindung nicht auf die zuvor beispielhaft genannten Lösemittel beschränkt.

Die Reaktionstemperatur, bei welcher die Umsetzungen gemäß den vorstehenden Ausführungsformen durchgeführt werden können, kann in weiten Bereichen variieren. Beispielsweise können die Umsetzungen bei einer Temperatur von 0 bis 100 °C, vorzugsweise 20 bis 70 °C, durchgeführt werden.

Der Umsetzungen der vorliegenden Erfindungen werden im Allgemeinen unter Normaldruck durchgeführt. Es ist jedoch auch möglich, unter erhöhtem Druck oder vermindertem Druck - im Allgemeinen zwischen 0,1 bar und 10 bar - zu arbeiten.

Die Verfahren zur Herstellung der erfindungsgemäßen N-Azinyl-N'-pyridylsulfonyl-harnstoffe der allgemeinen Formel (I) werden gegebenenfalls in Gegenwart eines basischen Reaktionshilfsmittels durchgeführt.

Als solche kommen alle üblichen anorganischen oder organischen Basen infrage. Hierzu gehören beispielsweise Alkalimetall- oder Erdalkalimetallhydride, -hydroxide, -amide, -alkoholate, -acetate, -carbonate oder -hydrogencarbonate, wie beispielsweise Lithium-, Natrium-, Kalium- oder Calciumhydrid, Lithium-, Natrium- oder Kaliumamid, Natrium- oder Kaliummethylat, Natrium- oder Kaliumethylat, Natrium- oder Kaliumpropylat, Aluminiumisopropylat, Natrium- oder Kalium-tert.-butylat, Natrium- oder Kaliumhydroxid, Ammoniumhydroxid, Natrium-, Kalium- oder Calciumacetat, Ammoniumacetat, Natrium-, Kalium- oder Calciumcarbonat, Ammoniumcarbonat, Natrium- oder Kaliumhydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyldicyclohexylamin, N,N-Dimethylanilin, N,N-Dimethylbenzylamin, Pyridin, 2-Methyl-, 3-Methyl- und 4-Methylpyridin, 2,4-Dimethyl-, 2,6-Dimethyl-, 3,4-Dimethyl- und 3,5-Dimethylpyridin, 5-Ethyl-2-methyl-pyridin, N-Methylpyridin, 4-(N,N-Dimethylamino)-pyridin, Diazabicyclooctan (DABCO), Diazabicyclononen (DBN) oder Diazabicycloundecen (DBU). Intermediate

Weiterer Gegenstand der vorliegenden Erfindung sind auch bestimmte Intermediate, welche gemäß den oben dargestellten Synthesewegen bei der Herstellung der erfindungsgemäßen Verbindungen der allgemeinen Formel (I) durchlaufen werden.

Demnach betrifft die vorliegende Erfindung in einer ersten Ausführungsform der Intermediate auch Verbindungen der allgemeinen Formel (II) 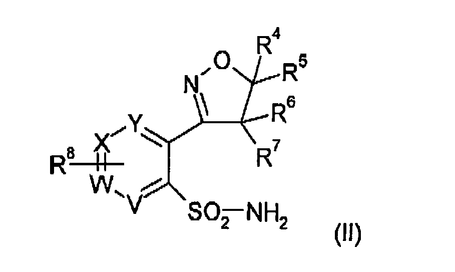 in welchen die Reste V, W, X, Y, R⁴, R⁵, R⁶, R⁷ und R⁸ die bereits weiter oben angegebenen allgemeinen, bevorzugten und besonders bevorzugten Bedeutungen aufweisen.

Weiterer Gegenstand der vorliegenden Erfindung sind in einer zweiten Ausführungsform der Intermediate auch Verbindungen der allgemeinen Formel (IV) 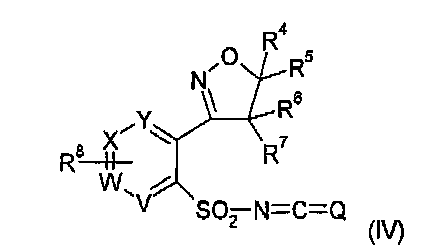 in welchen die Reste V, W, X, Y, R⁴, R⁵, R⁶, R⁷ , R⁸ und Q die bereits weiter oben angegebenen allgemeinen, bevorzugten und besonders bevorzugten Bedeutungen aufweisen.

Weiterer Gegenstand der vorliegenden Erfindung sind in einer dritten Ausführungsform der Intermediate auch Verbindungen der allgemeinen Formel (X) 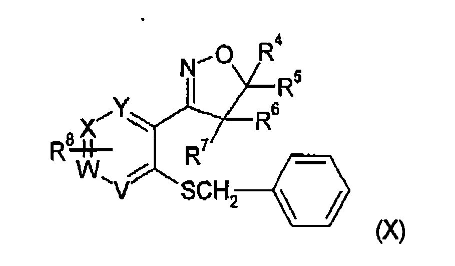 in welchen die Reste V, W, X, Y, R⁴, R⁵, R⁶, R⁷ und R⁸ die bereits weiter oben angegebenen allgemeinen, bevorzugten und besonders bevorzugten Bedeutungen aufweisen.

Weiterer Gegenstand der vorliegenden Erfindung sind in einer vierten Ausführungsform der Intermediate auch Verbindungen der allgemeinen Formel (XI) 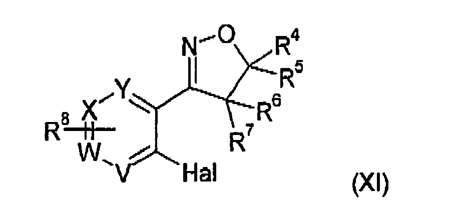 in welchen die Reste V, W, X, Y, R⁴, R⁵, R⁶, R⁷, R⁸ und Hal die bereits weiter oben angegebenen allgemeinen, bevorzugten und besonders bevorzugten Bedeutungen aufweisen.

Weiterer Gegenstand der vorliegenden Erfindung sind in einer fünften Ausführungsform der Intermediate auch Verbindungen der allgemeinen Formel (VIII) 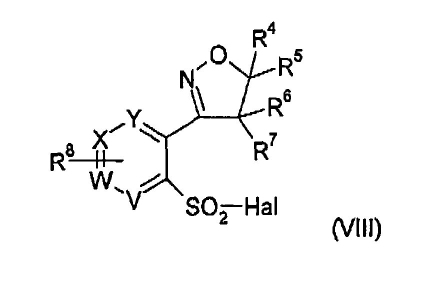 in welchen die Reste V, W, X, Y, R⁴, R⁵, R⁶, R⁷, R⁸ und Hal die bereits weiter oben angegebenen allgemeinen, bevorzugten und besonders bevorzugten Bedeutungen aufweisen.

Weiterer Gegenstand der vorliegenden Erfindung sind in einer sechsten Ausführungsform der Intermediate auch Verbindungen der allgemeinen Formel (VI) 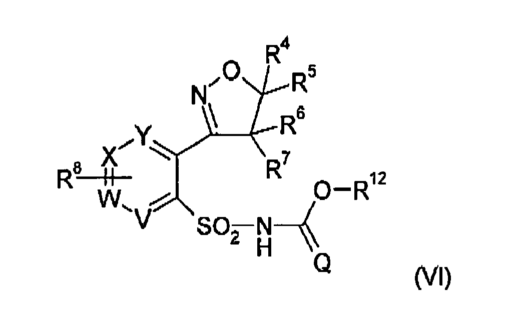 in welchen die Reste V, W, X, Y, R⁴, R⁵, R⁶, R⁷, R⁸ R¹² und Q die bereits weiter oben angegebenen allgemeinen, bevorzugten und besonders bevorzugten Bedeutungen aufweisen.

Bibliotheken aus Verbindungen der Formel (I) und/oder deren Salzen, die nach den oben genannten Reaktionen synthetisiert werden können, können auch in paralielisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur paralielisierten Reaktionsdurchführung und Aufarbeitung können eine Reihe von im Handel erhältlichen Geräten verwendet werden, beispielsweise Calypso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen-unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland. Die Reaktionen können beispielsweise auch mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Mit den äußeren Bedingungen wie Temperatur, Feuchtigkeit, der Art des verwendeten Herbizids, variiert u.a. die erforderliche Aufwandmenge der Verbindungen der Formel (I). Sie kann innerhalb weiter Grenzen schwanken, z.B. zwischen 0,001 und 10000 g/ha oder mehr Aktivsubstanz, vorzugsweise liegt sie jedoch zwischen 0,5 und 5000 g/ha, bevorzugt zwischen 0.5 und 1000 g/ha und ganz besonders bevorzugt zwischen 0.5 und 500 g/ha.

Die erfindungsgemäßen Verbindungen der Formel (I) und deren Salze, im Folgenden synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotyle Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschte Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagem hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickeinden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259).
- transgene Kulturpflanzen mit modifizierter Fettsäurezusammensetzung (WO 91/013972).
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461)
- gentechnisch veränderte Pflanzen mit reduzierter Photorespiration, die höhere Erträge und höhere Stresstoleranz aufweisen (EP 0305398)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nueleinsäutemolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents, and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Athylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenem, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischem unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt. Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenem, Düngemitteln und/oder Wachstumsregulatoren kombiniert eingesetzt werden, z. B. als Fertigformulierung oder als Tankmischungen. Als Kombinationspartner für die erfindungsgemäßen Wirkstoffe der allgemeinen Formel (I) in Mischformulierungen oder im Tank-Mix sind beispielsweise bekannte Wirkstoffe, die auf einer Inhibition von beispielsweise

Acetolactat-Synthase, Acetyl-Coenzym-A-Carboxylase, PS I, PS II, HPPDO, Phytoene-Desaturase, Protoporphyrinogen-Oxidase, Glutamine-Synthetase, 5-Enolpyruvylshikimat-3-phosphat-Synthetase oder der Cellulosebiosynthese beruhen, einsetzbar. Solche Verbindungen und auch andere einsetzbare Verbindungen mit teilwiese unbekanntem oder anderem Wirkungsmechanismus sind z. B. in Weed Research 26, 441-445 (1986), oder "The Pesticide Manual", 11. Auflage 1997 (im Folgenden auch kurz "PM") und 12. Auflage 2000, The British Crop Protection Council and the Royal Soc. of Chemistry (Herausgeber), und dort zitierter Literatur beschrieben. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):

acetochlor; acifluorfen(-sodium); aclonifen; AKH 7088, d.h. [[[1-[5-[2-Chloro-4-(trifluormethyl)-phenoxy]-2-nitrophenyl]-2-methoxyethylidene]-amino]-oxy]-essigsäure und -essigsäuremethylester; acrolein; alachlor; alloxydim(-sodium); ametryn; amicarbazone, amidochlor, amidosulfuron; aminopyralid, amitrol; AMS, d.h. Ammoniumsulfamat; anilofos; asulam; atraton; atrazin; azafenidin, azimsulfuron (DPX-A8947); aziprotryn; barban; BAS 516 H, d.h. 5-Fluor-2-phenyl-4H-3,1-benzoxazin-4-on; BCPC; beflubutamid, benazolin(-ethyl); benfluralin; benfuresate; bensulfuron(-methyl); bensulide; bentazone; benzfendizone; benzobicyclon, benzofenap; benzofluor; benzoylprop(-ethyl); benzthiazuron; bifenox; bialaphos; bifenox; bispyribac(-sodium), borax; bromacil; bromobutide; bromofenoxim; bromoxynil; bromuron; buminafos; busoxinone; butachlor; butafenacil, butamifos; butenachlor; buthidazole; butralin; butroxydim, butylate; cacodylic acid; calcium chlorate; cafenstrole (CH-900); carbetamide; carfentrazone(-ethyl); caloxydim, CDAA, d.h. 2-Chlor-N,N-di-2-propenylacetamid; CDEC, d.h. Diethyldithiocarbaminsäure-2-chlorallylester; chlorflurenol (-methyl); chlomethoxyfen; clethodim; clomeprop; chloramben; chlorazifop-butyl, chlormesulon; chlorbromuron; chlorbufam; chlorfenac; chlorflurecol-methyl; chloridazon; chlorimuron(-ethyl); chloroacetic acid; chlomitrofen; chlorotoluron; chloroxuron; chlorpropham; chlorsulfuron; chlorthal(-dimethyl); chlorthiamid; chlortoluron, cinidon(-methyl und -ethyl), cinmethylin; cinosulfuron; cisanilide; clefoxydim, clethodim; clodinafop und dessen Esterderivate (z. B. clodinafop-propargyl); clomazone; clomeprop; cloproxydim; clopyralid; clopyrasulfuron(-methyl); cloransulam(-methyl), cresol; cumyluron (JC 940); cyanamide; cyanazine; cycloate; cyclosulfamuron (AC 104); cycloxydim; cycluron; cyhalofop und dessen Esterderivate (z. B. Butylester, DEH-112); cyperquat; cyprazine; cyprazole; daimuron; 2,4-D, 2,4-DB, 3,4-DA, 3,4-DB, 2,4-DEB, dalapon; dazomed; desmedipham; desmetryn; di-allate; dicamba; dichlobenil; ortho-dichlorobenzene; para-dichlorobenzene; dichlorprop; dichlorprop-P; diclofop und dessen Ester wie diclofopmethyl; diclosulam, diethatyl(-ethyl); difenoxuron; difenzoquat; difenzoquat-methylsulphate; diflufenican; diflufenzopyr, dimefuron; dimepiperate, dimethachlor; dimethametryn; dimethenamid (SAN-582H); dimethenamid-P; dimethazone, dimexyflam, dimethipin; diemethylarsinic acid; dinitramine; dinoseb; dinoterb; diphenamid; dipropetryn; diquat; diquat-dibromide; dithiopyr; diuron; DNOC; 3,4-DP; DSMA; EBEP; eglinazine-ethyl; EL77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron(-methyl); ethidimuron; ethiozin; ethofumesate; ethoxyfen und dessen Ester (z. B. Ethylester, HN-252); ethoxysulfuron, etobenzanid (HW 52); F5231, d.h. N-[2-Chlor-4-fluor-6-[4-(3-fluorpropyl)-4,5-dihydro-5-oxo-1H-tetrazol-1-yl]-phenyl]-ethansulfonamid; fenoprop; fenoxan, fenoxaprop und fenoxaprop-P sowie deren Ester, z. B. fenoxaprop-P-ethyl und fenoxaprop-ethyl; fenoxydim; fentrazamide, fenuron; ferrous-sulphate; flamprop(-methyl oder -isopropyl oder - isopropyl-L); flazasulfuron; floazulate, florasulam, fluazifop und fluazffop-P und deren Ester, z. B. fluazifop-butyl und fluazifop-P-butyl; fluazolate; flucarbazone(-sodium), flucetosulfuron; fluchloralin; flufenacet; flufenpyr(-ethyl); flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanate, flupyrsulfuron(-methyl oder -sodium), flurenol(-butyl), fluridone; flurochloridone; fluroxypyr(-meptyl); flurprimidol; flurtamone; fluthiacet(-methyl) (KIH-9201); fluthiamide; fomesafen; foramsulfuron; fosamine; furyloxyfen; glufosinate(-ammonium); glyphosate(-isopropylammonium); halosafen; halosulfuron(-methyl) und dessen Ester (z. B. Methylester, NC-319); haloxyfop und dessen Ester; haloxyfop-P (= R-haloxyfop) und dessen Ester; HC-252; hexazinone; imazamethabenz(-methyl); imazapyr; imazaquin und Salze wie das Ammoniumsalz; imazamethapyr, imazamox, imazapic, imazethamethapyr; imazethapyr; imazosulfuron; indanofan, iodomethane; iodosulfuron(methylsodium); ioxynil; isocarbamid; isopropalin; isoproturon; isouron; isoxaben; isoxachlortole, isoxaflutole, isoxapyrifop; karbutilate; lactofen; lenacil; linuron; MAA; MAMA; MCPA; MCPA-2-ethylhexyl; MCPA-thioethyl; MCPB; mecoprop; mecoprop-P; mefenacet; mefluidid; mesosulfuron(-methyl); mesotrione, metamifop; metamitron; metazachlor; methabenzthiazuron; metham; methazole; methoxyphenone; methylarsonic acid; methyldymron; methyl isothiocyanate; metabenzuron, metamifop; methobenzuron; metobromuron; (alpha-)metolachlor, S- metolachlor; metosulam (XRD 511); metoxuron; metribuzin; metsulfuron-methyl; MK-616; MH; molinate; monalide; monocarbamide dihydrogensulfate; monolinuron; monuron; monosulfuron; MSMA; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl] -2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; nonanoic acid; norflurazon; oleic acid (fatty acid); orbencarb; orthosulfamuron; oryzalin; oxadiargyl (RP-020630); oxadiazon; oxasulfuron, oxaziclomefone, oxyfluorfen; paraquat; paraquat-dichloride; pebulate; pelargonic acid, pendimethalin; penoxsulam; pentachlorophenol; pentanochlor; pentoxazone, perfluidone; phenisopham; phenmedipham(ethyl); pethoxamid: picloram; picolinafen, pinoxaden, piperophos; piributicarb; pirifenop-butyl; pretilachlor; primisulfuron(-methyl); potassium arsenite; potassium azide; procarbazone-(sodium), procyazine; prodiamine; profluazol; profluralin; profoxydim; proglinazine(-ethyl), prometon; prometryn; propachlor; propanil; propaquizafop und dessen Ester; propazine; propham; propisochlor; propoxycarbazone(-sodium) (BAY MKH 6561); propyzamide; prosulfalin; prosulfocarb; prosulfuron (CGA-152005); prynachlor; pyraclonil; pyraflufen(-ethyl), pyrasulfotole; pyrazolinate; pyrazon; pyrazosulfuron(-ethyl); pyrazoxyfen; pyribambenz-isopropyl; pyribenzoxim, pyributicarb, pyridafol, pyridate; pyriftalid; pyrimidobac(-methyl), pyrimisulfan, pyrithiobac(-sodium) (KIH-2031); pyroxasulfone; pyroxofop und dessen Ester (z. B. Propargylester); pyroxsulam (triflosulam); quinclorac; quinmerac; quinoclamine, quinofop und dessen Esterderivate, quizalofop und quizalofop-P und deren Esterderivate z. B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; saflufenacil (CAS-RN: 372137-35-4); secbumeton; sethoxydim; siduron; simazine; simetryn; SN 106279, d.h. 2-[[7-[2-Chlor-4-(trifluor-methyl)-phenoxy]-2-naphthalenyl]-oxyl-propansäure und -methylester; SMA; sodium arsenite; sodium azide; sodium chlorate; sulcotrione, sulfentrazon (FMC-97285, F-6285); sulfazuron; sulfometuron(-methyl); sulfosate (ICI-A0224); sulfosulfuron, 2,3,6-TBA; TCA(sodium); tebutam (GCP-5544); tebuthiuron; tefuryltrione, tembotrione, tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850): thiafluamide, thiazafluron; thiazopyr (Mon-13200); thidiazimin (SN-24085); thiencarbazonemethyl, thifensulfuron(-methy); thiobencarb; tiocarbazil; tralkoxydim; tri-allate; triasulfuron; triaziflam, triazofenamide; tribenuron(-methyl); tricamba; triclopyr; tridiphane; trietazine; trifloxysulfuron(sodium); trifluralin; triflusulfuron-methyl und Ester (z. B. Methylester, DPX-66037); trihydroxytriazine; trimeturon; tritosulfuron; tropramezone; tsitodef; vemolate; [3-[2-chloro4-fluoro-5-(1-methyl-6-trifluormethyl-2,4-dioxo-1,2,3,4-tetrahydropyrimidin-3-yl)phenoxy]-2-pyridyloxy]acetic acid ethyl ester; WL 110547, d.h. 5-Phenoxy-1-[3-(trfftuormethyl)-phenyl]-1H-tetrazol; UBH-509; D-489; LS 82-556, d.h. [(S)-3-N-(methylbenzyl)carbamoyl-5- propionyl-2,6-lutidine]; KPP-300; NC-324; NC-330; KH-218; DPX-N8189; SC-0774; DOWCO-535; DK-8910; V-53482; PP-600; MBH-001; ET-751, d. h Ethyl-[2-Chlor-5-(4-chlor-5-difluormethoxy-1-methyl-1H-pyrazol-3-yl)-4-fluor-phenoxy]-acetat; KIH-6127, d.h. Pyriminobac-methyl; KIH-2023, d.h. Bispyribac-Natrium; und SYP-249, d.h Ethyl 2-{2-nitro-5-[(2-chloro-4-trifluoromethyl)phanoxy]-benzoxy}-3-methyl-3-butenoate; SYN-523.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit wieteren Pestiziden in Frage:

Die Safener sind vorzugsweise ausgewählt aus der Gruppe bestehend aus:

S1) Verbindungen der Formel (S1), 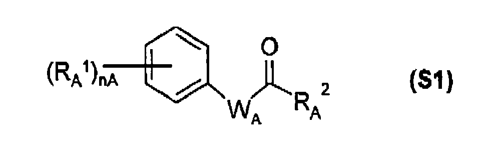 wobei die Symbole und Indizes folgende Bedeutungen haben:
- n_{A}: ist eine natürliche Zahl von 0 bis 5, vorzugsweise 0 bis 3;
- R_{A}¹: ist Halogen, (C₁-C₄)Alkyl, (C₁-C₄)Alkoxy, Nitro oder (C₁-C₄)Haloalkyl;
- W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O wobei mindestens ein N-Atom und höchstens ein O-Atom im Ring enthalten ist, vorzugsweise ein Rest aus der Gruppe (W_{A}¹) bis (W_{A}⁴),

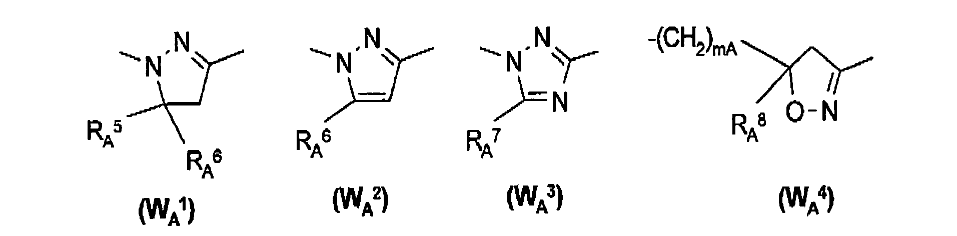

- m_{A}: ist 0 oder 1;
- R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Hetero-atomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls sub- stituiertes Phenyl substituiert ist, vorzugsweise ein Rest der Formel OR_{A}³, NHR_{A}⁴ oder N(CH₃)₂, insbesondere der Formel OR_{A}³;
- R_{A}³: ist Wasserstoff oder ein unsubstituierter oder substituierter aliphatischer Kohlenwasserstoffrest, vorzugsweise mit insgesamt 1 bis 18 C-Atomen;
- R_{A}⁴: ist Wasserstoff, (C₁-C₆)Alkyl, (C₁-C₆)Alkoxy oder substituiertes oder unsubstituiertes Phenyl;
- R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
- R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
vorzugsweise:
a) Verbindungen vom Typ der Dichlorphenylpyrazolin-3-carbonsäure (S1^{a}), vorzugsweise Verbindungen wie
   1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91107874 beschrieben sind;
b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethyl-ester (S1-2), 1-(2,4-Dichlorphenyl)-5-isopropyl-pyrazot-3-carbonsäureethyl-ester (S1-3), 1-(2,4-Dichlorphenyl)-5-(1,1-dimethyl-ethyl)pyrazol-3-carbon-säureethyl-ester (S1-4) und verwandte Verbindungen, wie sie in EP-A-333 131 und EP-A-269 806 beschrieben sind;
c) Derivate der 1,5-Diphenyfpyrazol-3-carbonsäure (S1^{c}), vorzugsweise Verbindungen wie 1-(2,4-Dichlorphenyl)-5-phenylpyrazol-3-carbonsäureethyl-ester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäure-ethylester (S1-7), und verwandte Verbindungen wie sie in EP-A-174 562 und EP-A-346 620 beschrieben sind;
e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.

S2) Chinolinderivate der Formel (S2), 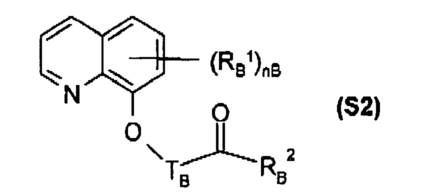 wobei die Symbole und Indizes folgende Bedeutungen haben:
- R_{B}¹: ist Halogen, (C₁-C₄)Alkyl, (C₁-C₄)Alkoxy, Nitro oder (C₁-C₄)Haloalkyl;
- n_{B}: ist eine natürliche Zahl von 0 bis 5, vorzugsweise 0 bis 3;
- R_{B}²: ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³_{;}
- R_{B}³: ist Wasserstoff oder ein unsubstituierter oder substituierter aliphatischer Kohlenwasserstoffrest, vorzugsweise mit insgesamt 1 bis 18 C-Atomen;
- R_{B}⁴: ist Wasserstoff, (C₁-C₆)Alkyl, (C₁-C₆)Alkoxy oder substituiertes oder unsubstituiertes Phenyl;
- T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
vorzugsweise:
a) Verbindungen vom Typ der 8-Chinolinoxyessigsäure (S2^{a}), vorzugsweise (5-Chlor-8-chinolinoxy)essigsäure-(1-methylhexyl)ester ("Cloquintocetmexyl") (S2-1),
   (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinofinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen- , Ammonium-, quartäre Ammonium-, Sulfonium-, oder Phosphoniumsalze wie sie in der WO-A-2002/34048 beschrieben sind;
b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.

S3) Verbindungen der Formel (S3) 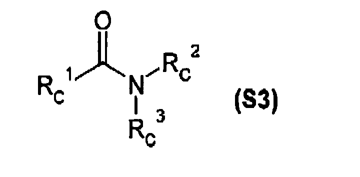 wobei die Symbole und Indizes folgende Bedeutungen haben:
- R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
- R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
vorzugsweise:
Wirkstoffe vom Typ der Dichloracetamide, die häufig als Vorauflaufsafener (bodenwirksame Safener) angewendet werden, wie z. B.
"Dichlormid" (N,N-Diallyl-2,2-dichloracetamid) (S3-1),
"R-29148" (3-Dichloracetyl-2,2,5-trimethyl-1,3-oxazolidin) der Firma Stauffer (S3-2),
"R-28725" (3-Dichloracetyl-2,2,-dimethyl-1,3-oxazolidin) der Firma Stauffer (S3-3),
"Benoxacor" (4-Dichloracetyl-3,4-dihydro-3-methyl-2H-1,4-benzoxazin) (S3-4),
"PPG-1292" (N-Allyl-N-[(1,3-dioxolan-2-yl)-methyl]-dichloracetamid) der Firma PPG Industries (S3-5),
"DKA-24" (N-Allyl-N-[(allylaminocarbonyl)methyl]-dichloracetamid) der Firma Sagro-Chem (S3-6),
"AD-67" oder "MON 4660" (3-Dichloracetyl-1-oxa-3-aza-spiro[4,5]decan) der Firma Nitrokemia bzw. Monsanto (S3-7),
"TI-35" (1-Dichloracetyl-azepan) der Firma TRI-Chemical RT (S3-8),
"Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
"Furilazol" oder "MON 13900" (RS)-3-Dichloracetyl-5-(2-furyl)-2,2-dimethyl-oxazolidin) (S3-10); sowie dessen (R)-Isomer (S3-11).

S4) N-Acylsulfonamide der Formel (S4) und ihre Salze, 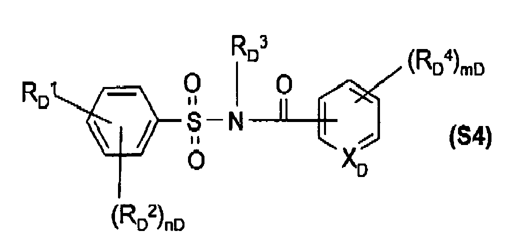 worin die Symbole und Indizes folgende Bedeutungen haben:
- X_{D}: ist CH oder N;
- R_{D}¹: ist CO-NR_{D}⁵R_{D}⁶ oder NHCO-R_{D}⁷;
- R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
- R_{D}³: ist Wasserstoff, (C₁-C₄)Alkyl, (C₂-C₄)Alkenyl oder (C₂-C₄)Alkinyl;
- R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
- R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
- R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
- R_{D}⁵ und R_{D}⁶: gemeinsam mit dem dem sie tragenden Stickstoffatom einen Pyrrolidinyl- oder Piperidinyl-Rest bilden;
- R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
- n_{D}: ist 0, 1 oder 2;
- m_{D}: ist 1 oder 2;
- v_{D}: ist 0, 1, 2 oder 3;
davon bevorzugt sind Verbindungen von Typ der N-Acylsulfonamide, z.B. der nachfolgenden Formel (S4^{a}), die z. B. bekannt sind aus WO-A-97/45016 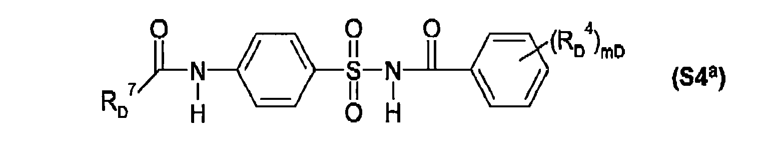 worin
- R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
- R_{D}⁴: Halogen, (C₁-C₄)Alkyl, (C₁-C₄)Alkoxy, CF₃;
- m_{D}: 1 oder 2:
- v_{D}: ist 0, 1, 2 oder 3 bedeutet;
sowie

Acylsulfamoylbenzoesäureamide, z.B. der nachfolgenden Formel (S4^{b}), die z.B. bekannt sind aus WO-A-99/16744, 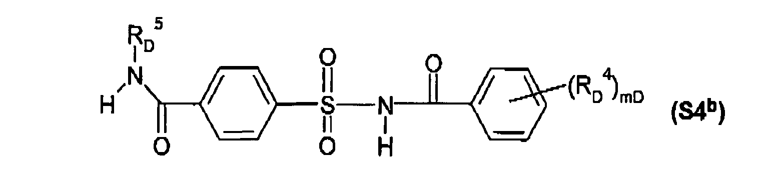 z.B. solche worin
- R_{D}⁵ =: Cyclopropyl und (R_{D}⁴) = 2-OMe ist("Cyprosulfamide", S4-1),
- R_{D}⁵=: Cyclopropyl und (R_{D}⁴) = 5-Cl-2-OMe ist (S4-2),
- R_{D}⁵ =: Ethyl und (R_{D}⁴)= 2-OMe ist(S4-3),
- R_{D}⁵ =: Isopropyl und (R_{D}⁴) = 5-Cl-2-OMe ist (S4-4) und
- R_{D}⁵=: Isopropyl und (R_{D}⁴) = 2-OMe ist (S4-5).
sowie

Verbindungen vom Typ der N-Acylsulfamoylphenylharnstoffe der Formel (S4^{c}), die z.B. bekannt sind aus der EP-A-365484, 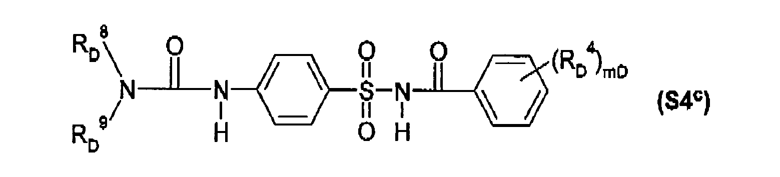 worin
- R_{D}⁸: unabhängig voneinander Wasserstoff, (C₁-C₈)Alkyl, (C₃-C₈)Cycloalkyl, (C₃-C₆)Alkenyl, (C₃-C₆)Alkinyl,
- R_{D}⁴: Halogen, (C₁-C₄)Alkyl, (C₁-C₄)Alkoxy, CF₃
- m_{D}: 1 oder 2 bedeutet;
beispielsweise
1-[4-(N-2-Methoxybenzoylsulfamoyl)phenyl]-3-mothylharnstoff,
1-[4-(N-2-Methoxybenzoylsulfamoyl)phenyl]-3,3-dimethylhamstoff,
1-[4-(N-4,5-Dimethylbenzoyisulfämoyl)phenyl]-3-methylharnstoff,

S5) Wirkstoffe aus der Klasse der Hydroxyaromaten und der aromatischaliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.

S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.

S7) Verbindungen der Formel (S7),wie sie in der WO-A-1998/38856 beschrieben sind 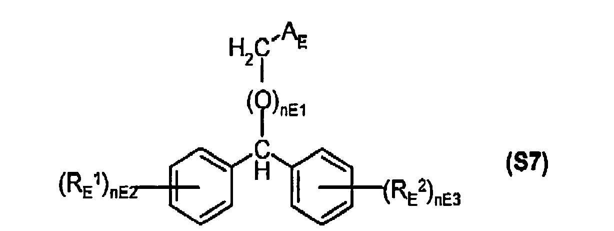 worin die Symbole und Indizes folgende Bedeutungen haben:
- R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
- A_{E}: ist COOR_{E}³ oder COSR_{E}⁴
- R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
- n_{E}¹: ist 0 oder 1
- n_{E}² , n_{E}³: sind unabhängig voneinander 0, 1 oder 2,
vorzugsweise:
Diphenylmethoxyessigsäure,
Diphenyfmethoxyessigsäureethylester,
Diphenylmethoxyessigsäuremethylester (CAS-Reg.Nr. 41858-19-9) (S7-1).

S8) Verbindungen der Formel (S8),wie sie in der WO-A-98/27049 beschrieben sind 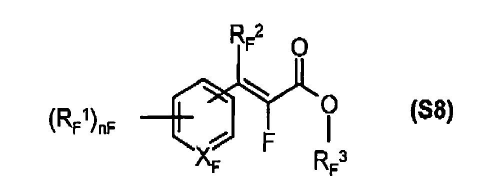 worin
- X_{F}: CH oder N,
- n_{F}: für den Fall, dass X_{F}=N ist, eine ganze Zahl von 0 bis 4 und für den Fall, dass X_{F}=CH ist, eine ganze Zahl von 0 bis 5 ,
- R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkyisulforlyl, (C₁-C₄)Alkoxycarbonyl, ggf. substituiertes. Phenyl, ggf. substituiertes Phenoxy,
- R_{F}²: Wasserstoff oder (C₁-C₄)Alkyl
- R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
vorzugsweise Verbindungen worin
- X_{F}: CH,
- n_{F}: eine ganze Zahl von 0 bis 2 ,
- R_{F}¹: Halogen, (C₁-C₄)Alkyl, (C₁-C₄)Haloalkyl, (C₁-C₄)Alkoxy, (C₁-C₄)Haloalkoxy,
- R_{F}²: Wasserstoff oder (C₁-C₄)Alkyl,
- R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
oder deren Salze.

S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylearbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.

S10) Verbindungen der Formeln (S10^{a}) oder (S10^{b})
wie sie in der WO-A-2007/023719 und WO-A-2007/023764 beschrieben sind 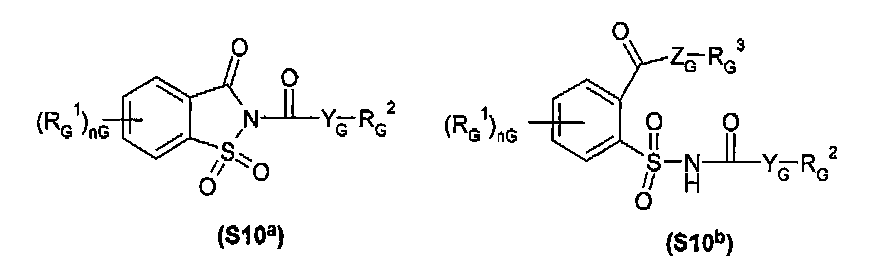 worin
- R_{G}¹: Halogen, (C₁-C₄)Alkyl, Methoxy, Nitro, Cyano, CF₃, OCF₃
- Y_{G}, Z_{G}: unabhängig voneinander O oder S,
- n_{G}: eine ganze Zahl von 0 bis 4,
- R_{G}²: (C₁-C₁₆)Alkyl, (C₂-C₆)Alkenyl, (C₃-C₆)Cycloalkyl, Aryl; Benzyl, Halogenbenzyl,
- R_{G}³: Wasserstoff oder (C₁-C₆)Alkyl bedeutet.

S11) Wirkstoffe vom Typ der Oxyimino-Verbindungen (S11), die als Saatbeizmittel bekannt sind, wie z. B.
"Oxabetrinil" ((Z)-1,3-Dioxolan-2-ylmethoxyimino(phenyl)acetonftril) (S11-1), das als Saatbeiz-Safener für Hirse gegen Schäden von Metolachlor bekannt ist,
"Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
"Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.

S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.

S13) Eine oder mehrere Verbindungen aus Gruppe (S13):
"Naphthalic anhydrid" (1,8-Naphthalindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist,
"MG-838" (CAS-Reg.Nr. 133993-74-5) (2-propenyl 1-oxa-4-azaspiro[4.5]decane-4-carbodithioate) (S13-6) der Firma Nitrokemia,
"Disutfoton" (O,O-Diethy) S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).

S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-hamstoff), das als Safener für Reis gegen Schäden des Herbizids Imazosulfuron bekannt ist,
"Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.

S15) Wirkstoffe, die vorrangig als Herbizide eingesetzt werden, jedoch auch Safenerwirkung auf Kulturpflanzen aufweisen, z.B.
(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl-3,6-dichlor-2-methoxybenzoat (Lactidichlor-ethyl).

Bevorzugt sind Herbizid-Safener-Kombinationen, enthaltend (A) eine herbizid wirksame Menge an einer oder mehrerer Verbindungen der Formel (I) oder deren Salzen, und (B) eine antidotische wirksame Menge an einem oder mehreren Safenem.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenem, die geeignet ist, die phytotoxische Wirkung von Pflanzenschutzmittelwirkstoffen (z. B. von Herbiziden) an Kulturpflanzen zu reduzieren.

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird ahand der nachfolgenden Beispiele näher erläutert, welche die Erfindung jedoch keinesfalls beschränken.

### A. Synthesebeispiele

1. N-[(4-Chlor-6-methoxy-pyrimidin-2-yl)carbamoyl]-3-(4,5-dihydroisoxazol-3-yl)pyridin-2-sulfonamide (la-1): 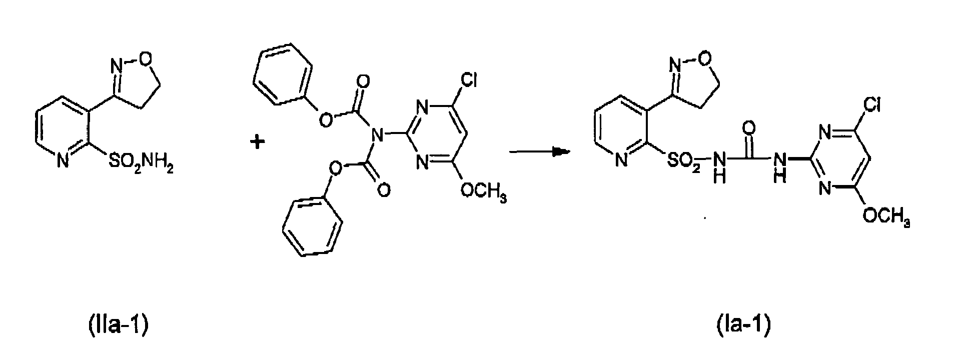 230 mg (1 mmol) 3-(4,5-Dihydroisoxazol-3-yl)pyddin-2-Sulfonamid (IIa-1) werden in 10 ml Acetonitril gelöst und mit 400 mg (1 mmol) Diphenyl-(4-chloro-6-methoxypyrimidin-2-yl)imidodicarbonat (vgl. WO 1996/022284) versetzt. Unter Rühren werden 160 mg (1 mmol) DBU (Diazabicyclo-undecen) zugetropft. Nach 1 Stunde Stehen bei 20 °C wird auf eine Mischung aus Methylenchlorid und wässriger Salzsäure (5 % ig) gegossen. Die organische Phase wird zweimal mit Wasser und einmal mit gesättigter Kochsalzlösung gewaschen. Nach Trocknen mit Magnesiumsulfat wird die organische Phase im Vakuum eingeengt. Der Rückstand wird in Diethylether verrieben und abgesaugt. Die erhaltenen Kristalle werden in Isopropanol verrührt, abgesaugt und getrocknet. Man erhält 200 mg (0.45 mmol) N-[(4-Chlor-6-methoxypyrimidin-2-yl)carbamoyl]-3-(4,5-dihydroisoxazol-3-yl)pyridin-2-sulfonamid (la-1) vom Reinheitsgehalt (HPLC) 92.8 %.
Die in den nachfolgenden Tabellen beschriebenen Verbindungen der allgemeinen Formel (I) erhält man gemäß oder analog zu dem oben beschriebenen Synthesebeispiel:

**Tabelle 1a: Verbindungen der Formel (Ia)**

| | | | | | | | | | | | |
|---|---|---|---|---|---|---|---|---|---|---|---|
| 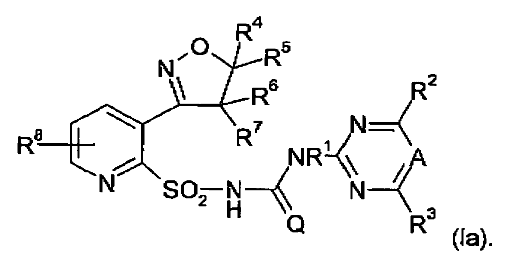 | | | | | | | | | | | |

| Bsp. Nr. | Phys. Daten | R¹ | R² | R³ | R⁴ | R⁶ | R⁵ | R⁷ | R⁸ | Q | A |
|---|---|---|---|---|---|---|---|---|---|---|---|
| Ia-1 | ¹H-NMR | H | Cl | OCH₃ | H | H | H | H | H | O | CH |
| Ia-2 | ¹H-NMR | H | OCH₃ | OCH₃ | H | H | H | H | H | O | CH |
| Ia-3 | ¹H-NMR | H | CH₃ | CH₃ | H | H | H | H | H | O | CH |
| Ia-4 | ¹H-NMR | H | SCH₃ | OCH₃ | H | H | H | H | H | O | CH |
| Ia-5 | ¹H-NMR | H | H | OCH₃ | H | H | H | H | H | O | CH |
| Ia-6 | ¹H-NMR | H | OCH₃ | OCH₃ | H | H | H | H | H | O | N |
| Ia-7 | ¹H-NMR | H | CH₃ | OCH₃ | H | H | H | H | H | O | N |
| Ia-8 | ¹H-NMR | H | N(CH₃)₂ | OCH₂CF₃ | H | H | H | H | H | O | N |
| Ia-9 | ¹H-NMR | H | CH₃ | OCH₃ | H | H | H | H | H | O | CH |
| Ia-10 | ¹H-NMR | H | OCH₃ | OCH₃ | CH(CH₃)₂ | H | H | H | H | O | CH |
| Ia-11 | ¹H-NMR | H | OCH₃ | OCH₃ | CH₃ | H | H | H | H | O | CH |
| Ia-12 | ¹H-NMR | H | CH₃ | OCH₃ | CH₃ | H | CH₃ | H | H | O | N |
| Ia-13 | ¹H-NMR | H | OCH₃ | OCH₃ | CH₃ | H | CH₃ | H | H | O | CH |
| Ia-14 | ¹H-NMR | H | Cl | OCH₃ | CH₃ | H | CH₃ | H | H | O | CH |
| Ia-15 | ¹H-NMR | H | CH₃ | OCH₃ | CH₃ | H | H | H | H | O | CH |
| Ia-16 | ¹H-NMR | H | Cl | OCH₃ | CH₃ | H | H | H | H | O | CH |
| Ia-17 | ¹H-NMR | H | CH₃ | OCH₃ | CH₂CH₂CH₃ | H | H | H | H | O | CH |
| Ia-18 | ¹H-NMR | H | CH₃ | OCH₃ | CH₂CH₃ | H | H | H | H | O | N |
| Ia-19 | ¹H-NMR | H | CH₃ | OCH₃ | CH₂CH₃ | H | H | H | H | O | CH |
| Ia-20 | ¹H-NMR | H | Cl | OCH₃ | CH₂CH₃ | H | H | H | H | O | CH |
| Ia-21 | ¹H-NMR | H | OCH₃ | OCH₃ | CH₂CH₂CH₃ | H | H | H | H | O | CH |
| Ia-22 | ¹H-NMR | H | CH₃ | OCH₃ | CH(CH₃)₂ | H | H | H | H | O | CH |
| Ia-23 | ¹H-NMR | H | CH₃ | OCH₃ | CH₂CH₂CH₃ | H | H | H | H | O | N |
| Ia-24 | ¹H-NMR | H | OCH₃ | OCH₃ | CH₂CH₃ | H | H | H | H | O | CH |
| Ia-25 | | H | OCH₃ | OCH₃ | H | H | H | H | H | S | CH |
| Ia-26 | | H | OCH₃ | OCH₃ | CH₂CH₂CH₂ | | H | H | H | O | CH |
| Ia-27 | | H | OCH₃ | OCH₃ | CH₂CH₂CH₂CH₂ | | H | H | H | O | CH |
| Ia-28 | | H | OCH₃ | OCH₃ | CH₂-O-CH₂ | | H | H | H | O | CH |
| Ia-29 | | H | OCH₃ | OCH₃ | CH₂-S-CH₂ | | H | H | H | O | CH |
| Ia-30 | | H | OCH₃ | OCH₃ | CH₂-O-CH₂CH₂ | | H | H | H | O | CH |
| Ia-31 | | H | OCH₃ | OCH₃ | CH₂-S-CH₂CH₂ | | H | H | H | O | CH |

| | | | | | | | | | | | |
|---|---|---|---|---|---|---|---|---|---|---|---|
| ¹H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) Ia-1: δ = 3.44 (t, 2H); 4.01 (s, 6H); 4.47 (t, 2H); 6.62 (s, 1H); 7.68 (dd, 1H); 7.99 (dd, 1 H); 8.39 (br. s, 1 H); 8.68 (dd, 1H); 12.2 (br. s, 1 H) ppm la-2: δ = 3.44 (t, 2H); 3.94 (s, 6H); 4.46 (t, 2H); 5.87 (s, 1H); 7.67 (dd, 1 H); 7.98 (dd, 1 H); 8.14 (br. s, 1H); 8.66 (dd, 1 H); 12.8 (br. s, 1 H) ppm Ia-3: δ= 2.42 (s, 6H); 3.45 (t, 2H); 4.46 (t, 2H); 6.90 (s, 1H); 7.66 (dd, 1H); 7.97 (dd, 1H); 8.06 (br. s, 1 H); 8.66 (dd, 1 H); 13.3 (br. s, 1 H) ppm la-4: δ = 2.55 (s, 3H); 3.45 (t, 2H); 3.94 (s, 3H); 4.47 (t, 2H); 6.42 (s, 1H); 7.67 (dd, 1 H); 7.98 (dd, 1 H); 8.18 (br. s, 1 H); 8.67 (dd, 1H); 12.9 (br. s, 1 H) ppm la-5: δ = 3.45 (t, 2H); 3.97 (s, 3H); 4.46 (t, 2H); 6.54 (d, 1H); 7.66 (dd, 1 H); 7.97 (dd, 1H); 8.1-8.4 (br. 1 H); 8.29 (d, 1H); 8.66 (dd, 1H); 13.1 (br. s, 1 H) ppm Ia-6: δ = 3.44 (t, 2H); 4.03 (s, 6H); 4.47 (t, 2H); 7.68 (dd, 1 H); 7.99 (dd, 1 H); 8.4 (br. s, 1H); 8.67 (dd, 1 H); 12.5 (br. s, 1H) ppm la-7: δ = 2.49 (s, 3H); 3.44 (t, 2H); 4.02 (s, 3H); 4.47 (t, 2H); 7.68 (dd, 1 H); 8.00 (dd, 1H); 8.4 (br., 1 H); 8.67 (dd, 1 H); 12.6 (br. s, 1 H) ppm Ia-8: δ = 3.16 (s, 3H); 3.19 (s, 3H); 3.44 (t, 2H); 4.47 (t, 2H); 4.87 (q, 2H); 7.67 (dd, 1 H); 7.98 (dd, 1H); 8.14 (br. s, 1H); 8.67 (dd, 1H); 12.9 (br. s, 1H) ppm la-9: δ = 3.43 (s, 3H); 3.45 (t, 2H); 3.95 (s, 3H); 4.46 (t, 2H); 6.40 (s, 1 H); 7.66 (dd, 1 H); 7.97 (dd, 1H); 8.0-8.25 (br., 1 H); 8.66 (dd, 1 H); 13.4 (br. s, 1 H) ppm la-10: δ = 0.95 (d, 3H); 1.01 (d, 3H); 3.18 (dd, 1H); 3.45 (dd, 1 H); 3.87 (m, 1H); 3.94 (s, 6H); 4.49 (m, 1H); 5.86 (s, 1H); 7.66 (dd, 1H); 7.97 (dd, 1H); 8.2 (br. s, 1 H); 8.65 (dd, 1 H); 12.8 (br. s, 1 H) ppm la-11: δ = 1.40 (d, 3H); 3.07 (dd, 1H); 3.54 (dd, 1H); 3.94 (s, 6H); 4.89 (m, 1H); 5.87 (s, 1 H); 7.66 (dd, 1H); 7.98 (dd, 1 H); 8.18 (br. s, 1 H); 8.65 (dd, 1H); 12.8 (br. s, 1 H) ppm Ia-12: δ =1.47 (s, 6H); 2.49 (s, 3H); 3.24 (s, 2H); 4.02 (s, 3H); 7.67 (dd, 1H); 7.99 (dd, 1 H); 8.45 (br. s, 1 H); 8.64 (dd, 1H); 12.6 (br. s, 1 H) ppm Ia-13: δ = 1.47 (s, 6H); 3.25 (s, 2H); 3.94 (s, 6H); 5.86 (s, 1H); 7.66 (dd, 1H); 7.99 (dd, 1 H); 8.16 (br. s, 1H); 8.64 (dd, 1H); 12.8 (br. s, 1 H) ppm la-14: δ = 1.47 (s, 6H); 3.25 (s, 2H); 4.00 (s, 3H); 6.61 (s, 1H); 7.67 (dd, 1H); 7.99 (dd, 1 H); 8.38 (br. s, 1H); 8.65 (dd, 1H); 12.2 (br. s, 1 H) ppm la-15: δ = 1.40 (d, 3H); 2.39 (s, 3H); 3.08 (dd, 1H); 3.55 (dd, 1H); 3.94 (s, 3H); 4.89 (m, 1H); 6.40 (s, 1H); 7.65 (dd, 1H); 7.96 (dd, 1H); 8.0-8.3 (br., 1H); 8.64 (dd, 1H); 13.4 (br., 1 H) ppm la-16: δ = 1.40 (d, 3H); 3.07 (dd, 1H); 3.54 (dd, 1H); 4.01 (s, 3H); 4.90 (m, 1H); 6.62 (s, 1H); 7.67 (dd, 1 H); 7.99 (dd, 1H); 8.40 (br. s, 1 H); 8.66 (dd, 1 H); 12.2 (br. s, 1H) ppm la-17: δ = 0.97 (t, 3H); 1.4-1.55 (m, 2H); 1.6-1.7 (m, 1H); 1.72-1.82 (m, 1H); 2.39 (s, 3H); 3.11 (dd, 1 H); 3.51 (dd, 1H); 3.95 (s, 3H); 4.75 (m, 1H); 6.40 (s, 1H); 7.65 (dd, 1 H); 7.97 (dd, 1H); 8.05-8.25 (br., 1 H); 8.64 (dd. 1 H); 13.4 (br. s, 1 H) ppm la-18: δ = 0.99 (t, 3H); 1.65-1.85 (m, 2H); 2.50 (s, 3H); 3.11 (dd, 1H); 3.49 (dd, 1H); 4.02 (s, 3H); 4.71 (m, 1H); 7.68 (dd, 1H); 7.99 (dd, 1H); 8.42 (br. s, 1H); 8.66 (dd, 1 H); 12.6 (br. s, 1H) ppm la-19: δ = 0.98 (t, 3H); 1.65-1.85 (m, 2H); 2.39 (s, 3H); 3.12 (dd, 1H); 3.51 (dd, 1H); 3.95 (s, 3H); 4.70 (m, 1 H); 6.40 (s, 1 H); 7.65 (dd, 1 H); 7.96 (dd, 1H); 8.13 (br. s, 1H); 8.65 (dd, 1 H); 13.4 (br. s, 1 H) ppm la-20: δ = 0.99 (t, 3H); 1.65-1.85 (m, 2H); 3.11 (dd, 1 H); 3.50 (dd, 1H); 4.01 (s, 3H); 4.70 (m, 1H); 6.62 (s, 1 H); 7.67 (dd, 1H); 7.98 (dd, 1 H); 8.39 (br. s, 1 H); 8.66 (dd, 1 H); 12.2 (br. s, 1 H) ppm la-21: δ = 0.98 (t, 3H); 1.40-1.55 (m, 2H); 1.6-1.7 (m, 1H); 1.7-1.85 (m. 1H); 3.11 (dd, 1 H); 3.51 (dd, 1 H); 3.94 (s, 6H); 4.75 (m, 1 H); 5.86 (s, 1H); 7.66 (dd, 1H); 7.97 (dd, 1H); 8.13 (br. s, 1 H); 8.64 (dd, 1 H); 12.8 (br. s, 1H) ppm la-22: δ = 0.95 (d, 3H); 1.00 (d, 3H); 2.39 (s, 3H); 3.18 (dd, 1 H); 3.46 (dd, 1H); 3.95 (s, 3H); 4.50 (m, 1H); 6.40 (s, 1H); 7.65 (dd, 1H); 7.96 (dd, 1 H); 8.05-8.25 (br., 1H); 8.65 (dd, 1H); 13.4 (br. s, 1H) ppm la-23: δ = 0.97 (t, 3H); 1.35-1.55 (m, 2H); 1.6-1.7 (m, 1H); 1.72-1.83 (m, 1 H); 2.50 (s, 3H); 3.10 (dd, 1 H); 3.50 (dd, 1 H); 4.02 (s. 3H); 4.78 (m, 1 H); 7.67 (dd, 1 H); 7.99 (dd, 1 H); 8.44 (br. s, 1 H); 8.65 (dd, 1 H); 12.6 (br. s, 1 H) ppm la-24: δ = 0.98 (t, 3H); 1.63-1.86 (m, 2H); 3.12 (dd, 1H); 3.50 (dd, 1H); 3.94 (s, 6H); 4.70 (m, 1H); 5.87 (s, 1 H); 7.66 (dd, 1 H); 7.97 (dd, 1 H); 8.15 (br. s, 1H); 8.65 (dd, 1 H); 12.8 (br. s, 1 H) ppm | | | | | | | | | | | |

**Tabelle 1b: Verbindungen der Formel (Ib)**

| | | | | | | | | | | |
|---|---|---|---|---|---|---|---|---|---|---|
| 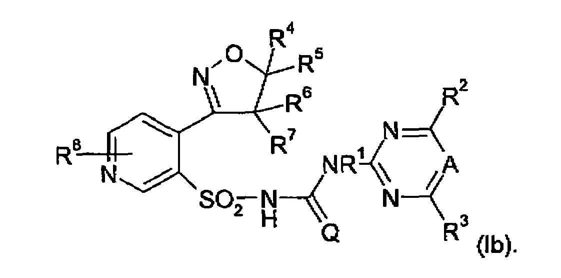 | | | | | | | | | | |

| Bsp. Nr. | R¹ | R² | R³ | R⁴ | R⁶ | R⁵ | R⁷ | R⁸ | Q | A |
|---|---|---|---|---|---|---|---|---|---|---|
| Ib-1 | H | Cl | OCH₃ | H | H | H | H | H | O | CH |
| Ib-2 | H | OCH₃ | OCH₃ | H | H | H | H | H | O | CH |
| Ib-3 | H | CH₃ | CH₃ | H | H | H | H | H | O | CH |
| Ib-4 | H | SCH₃ | OCH₃ | H | H | H | H | H | O | CH |
| Ib-5 | H | H | OCH₃ | H | H | H | H | H | O | CH |
| Ib-6 | H | OCH₃ | OCH₃ | H | H | H | H | H | O | N |
| Ib-7 | H | CH₃ | OCH₃ | H | H | H | H | H | O | N |
| Ib-8 | H | N(CH₃)₂ | OCH₂CF₃ | H | H | H | H | H | O | N |
| Ib-9 | H | CH₃ | OCH₃ | H | H | H | H | H | O | CH |
| Ib-10 | H | OCH₃ | OCH₃ | CH(CH₃)₂ | H | H | H | H | O | CH |
| Ib-11 | H | OCH₃ | OCH₃ | CH₃ | H | H | H | H | O | CH |
| Ib-12 | H | CH₃ | OCH₃ | CH₃ | H | CH₃ | H | H | O | N |
| Ib-13 | H | OCH₃ | OCH₃ | CH₃ | H | CH₃ | H | H | O | CH |
| Ib-14 | H | Cl | OCH₃ | CH₃ | H | CH₃ | H | H | O | CH |
| Ib-15 | H | CH₃ | OCH₃ | CH₃ | H | H | H | H | O | CH |
| Ib-16 | H | Cl | OCH₃ | CH₃ | H | H | H | H | O | CH |
| Ib-17 | H | CH₃ | OCH₃ | CH₂CH₂CH₃ | H | H | H | H | O | CH |
| Ib-18 | H | CH₃ | OCH₃ | CH₂CH₃ | H | H | H | H | O | N |
| Ib-19 | H | CH₃ | OCH₃ | CH₂CH₃ | H | H | H | H | O | CH |
| Ib-20 | H | Cl | OCH₃ | CH₂CH₃ | H | H | H | H | O | CH |
| Ib-21 | H | OCH₃ | OCH₃ | CH₂CH₂CH₃ | H | H | H | H O | O | CH |
| Ib-22 | H | CH₃ | OCH₃ | CH(CH₃)₂ | H | H | H | H | O | CH |
| Ib-23 | H | CH₃ | OCH₃ | CH₂CH₂CH₃ | H | H | H | H | O | N |
| Ib-24 | H | OCH₃ | OCH₃ | CH₂CH₃ | H | H | H | H | O | CH |
| Ib-25 | H | OCH₃ | OCH₃ | H | H | H | H | H | S | CH |
| Ib-26 | H | OCH₃ | OCH₃ | CH₂CH₂CH₂ | | H | H | H | O | CH |
| Ib-27 | H | OCH₃ | OCH₃ | CH₂CH₂CH₂CH₂ | | H | H | H | O | CH |
| Ib-28 | H | OCH₃ | OCH₃ | CH₂-O-CH₂ | | H | H | H | O | CH |
| Ib-29 | H | OCH₃ | OCH₃ | CH₂-S-CH₂ | | H | H | H | O | CH |
| Ib-30 | H | OCH₃ | OCH₃ | CH₂-O-CH₂CH₂ | | H | H | H | O | CH |
| Ib-31 | H | OCH₃ | OCH₃ | CH₂-S-CH₂CH₂ | | H | H | H | O | CH |

**Tabelle 1c: Verbindungen der Formel (Ic)**

| | | | | | | | | | | |
|---|---|---|---|---|---|---|---|---|---|---|
| 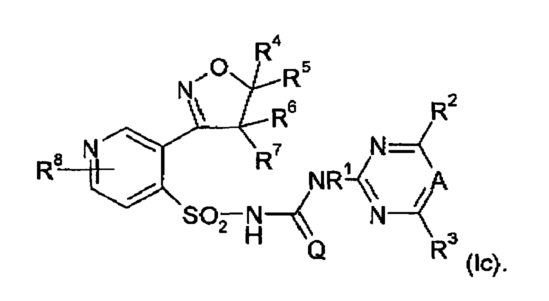 | | | | | | | | | | |

| Bsp. Nr. | R¹ | R² | R³ | R⁴ | R⁶ | R⁵ | R⁷ | R⁸ | Q | A |
|---|---|---|---|---|---|---|---|---|---|---|
| Ic-1 | H | OCH₃ | OCH₃ | H | H | H | H | H | O | CH |
| Ic-2 | H | Cl | OCH₃ | H | H | H | H | H | O | CH |
| Ic-3 | H | CH₃ | CH₃ | H | H | H | H | H | O | CH |
| Ic-4 | H | SCH₃ | OCH₃ | H | H | H | H | H | O | CH |
| Ic-5 | H | H | OCH₃ | H | H | H | H | H | O | CH |
| Ic-6 | H | OCH₃ | OCH₃ | H | H | H | H | H | O | N |
| Ic-7 | H | CH₃ | OCH₃ | H | H | H | H | H | O | N |
| Ic-8 | H | N(CH₃)₂ | OCH₂CF₃ | H | H | H | H | H | O | N |
| Ic-9 | H | CH₃ | OCH₃ | H | H | H | H | H | O | CH |
| Ic-10 | H | OCH₃ | OCH₃ | CH(CH₃)₂ | H | H | H | H | O | CH |
| Ic-11 | H | OCH₃ | OCH₃ | CH₃ | H | H | H | H | O | CH |
| Ic-12 | H | CH₃ | OCH₃ | CH₃ | H | CH₃ | H | H | O | N |
| Ic-13 | H | OCH₃ | OCH₃ | CH₃ | H | CH₃ | H | H | O | CH |
| Ic-14 | H | Cl | OCH₃ | CH₃ | H | CH₃ | H | H | O | CH |
| Ic-15 | H | CH₃ | OCH₃ | CH₃ | H | H | H | H | O | CH |
| Ic-16 | H | Cl | OCH₃ | CH₃ | H | H | H | H | O | CH |
| Ic-17 | H | CH₃ | OCH₃ | CH₂CH₂CH₃ | H | H | H | H | O | CH |
| Ic-18 | H | CH₃ | OCH₃ | CH₂CH₃ | H | H | H | H | O | N |
| Ic-19 | H | CH₃ | OCH₃ | CH₂CH₃ | H | H | H | H | O | CH |
| Ic-20 | H | Cl | OCH₃ | CH₂CH₃ | H | H | H | H | O | CH |
| Ic-21 | H | OCH₃ | OCH₃ | CH₂CH₂CH₃ | H | H | H | H | O | CH |
| Ic-22 | H | CH₃ | OCH₃ | CH(CH₃)₂ | H | H | H | H | O | CH |
| Ic-23 | H | CH₃ | OCH₃ | CH₂CH₂CH₃ | H | H | H | H | O | N |
| Ic-24 | H | OCH₃ | OCH₃ | CH₂CH₃ | H | H | H | H | O | CH |
| Ic-25 | H | OCH₃ | OCH₃ | H | H | H | H | H | S | CH |
| Ic-26 | H | OCH₃ | OCH₃ | CH₂CH₂CH₂ | | H | H | H | O | CH |
| Ic-27 | H | OCH₃ | OCH₃ | CH₂CH₂CH₂CH₂ | | H | H | H | O | CH |
| Ic-28 | H | OCH₃ | OCH₃ | CH₂-O-CH₂ | | H | H | H | O | CH |
| Ic-29 | H | OCH₃ | OCH₃ | CH₂-S-CH₂ | | H | H | H | O | CH |
| Ic-30 | H | OCH₃ | OCH₃ | CH₂-O-CH₂CH₂ | | H | H | H | O | CH |
| Ic-31 | H | OCH₃ | OCH₃ | CH₂-S-CH₂CH₂ | | H | H | H | O | CH |

**Tabelle 1d: Verbindungen der Formel (Id)**

| | | | | | | | | | | |
|---|---|---|---|---|---|---|---|---|---|---|
| 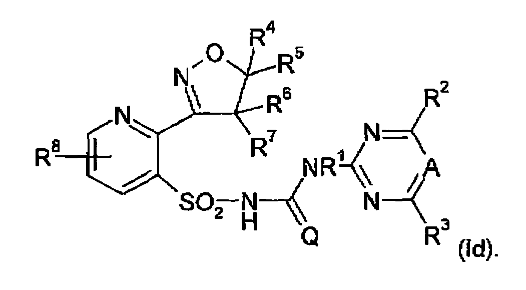 | | | | | | | | | | |

| Bsp. Nr. | R¹ | R² | R³ | R⁴ | R⁶ | R⁵ | R⁷ | R⁸ | Q | A |
|---|---|---|---|---|---|---|---|---|---|---|
| Id-1 | H | OCH₃ | OCH₃ | H | H | H | H | H | O | CH |
| Id-2 | H | Cl | OCH₃ | H | H | H | H | H | O | CH |
| Id-3 | H | CH₃ | CH₃ | H | H | H | H | H | O | CH |
| Id-4 | H | SCH₃ | OCH₃ | H | H | H | H | H | O | CH |
| Id-5 | H | H | OCH₃ | H | H | H | H | H | O | CH |
| Id-6 | H | OCH₃ | OCH₃ | H | H | H | H | H | O | N |
| Id-7 | H | CH₃ | OCH₃ | H | H | H | H | H | O | N |
| Id-8 | H | N(CH₃)₂ | OCH₂CF₃ | H | H | H | H | H | O | N |
| Id-9 | H | CH₃ | OCH₃ | H | H | H | H | H | O | CH |
| Id-10 | H | OCH₃ | OCH₃ | CN(CH₃)₂ | H | H | H | H | O | CH |
| Id-11 | H | OCH₃ | OCH₃ | CH₃ | H | H | H | H | O | CH |
| Id-12 | H | CH₃ | OCH₃ | CH₃ | H | CH₃ | H | H | O | N |
| Id-13 | H | OCH₃ | OCH₃ | CH₃ | H | CH₃ | H | H | O | CH |
| Id-14 | H | Cl | OCH₃ | CH₃ | H | CH₃ | H | H | O | CH |
| Id-15 | H | CH₃ | OCH₃ | CH₃ | H | H | H | H | O | CH |
| Id-16 | H | Cl | OCH₃ | CH₃ | H | H | H | H | O | CH |
| Id-17 | H | CH₃ | OCH₃ | CH₂CH₂CH₃ | H | H | H | H | O | CH |
| Id-18 | H | CH₃ | OCH₃ | CH₂CH₃ | H | H | H | H | O | N |
| Id-19 | H | CH₃ | OCH₃ | CH₂CH₃ | H | H | H | H | O | CH |
| Id-20 | H | Cl | OCH₃ | CH₂CH₃ | H | H | H | H | O | CH |
| Id-21 | H | OCH₃ | OCH₃ | CH₂CH₂CH₃ | H | H | H | H | O | CH |
| Id-22 | H | CH₃ | OCH₃ | CH(CH₃)₂ | H | H | H | H | O | CH |
| Id-23 | H | CH₃ | OCH₃ | CH₂CH₂CH₃ | H | H | H | H | O | N |
| Id-24 | H | OCH₃ | OCH₃ | CH₂CH₃ | H | H | H | H | O | CH |
| Id-25 | H | OCH₃ | OCH₃ | H | H | H | H | H | S | CH |
| Id-26 | H | OCH₃ | OCH₃ | CH₂CH₂CH₂ | | H | H | H | O | CH |
| Id-27 | H | OCH₃ | OCH₃ | CH₂CH₂CH₂CH₂ | | H | H | H | O | CH |
| Id-28 | H | OCH₃ | OCH₃ | CH₂-O-CH₂ | | H | H | H | O | CH |
| Id-29 | H | OCH₃ | OCH₃ | CH₂-S-CH₂ | | H | H | H | O | CH |
| Id-30 | H | OCH₃ | OCH₃ | CH₂-O-CH₂CH₂ | | H | H | H | O | CH |
| Id-31 | H | OCH₃ | OCH₃ | CH₂-S-CH₂CH₂ | | H | H | H | O | CH |

2. 3-(4,5-Dihydroisoxazol-3-yl)pyridin-2-sulfonamid (IIa-1) 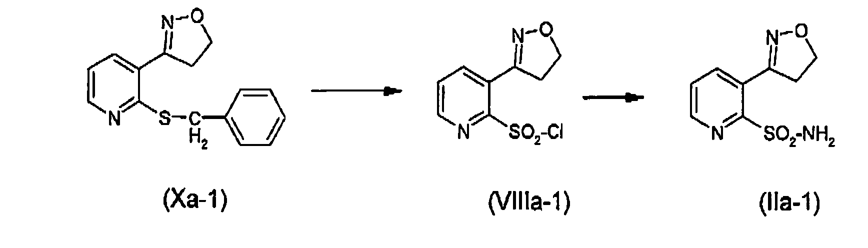 2.7 g (10 mmol) 2-(Benzylthio)-3-(4,5-dihydroisoxazol-3-yl)pyridin (Xa-1) werden in einer Mischung aus 50 ml Eisessig und 25 ml Wasser aufgenommen. In die entstandene Suspension wird bei 0 °C Chlorgas eingeleitet, bis eine schwache Gelbfärbung eintritt. Die klare Lösung wird dreimal mit Methylenchlorid extrahiert. Die vereinigten organischen Phasen werden mit Wasser und Kochsalzlösung gewaschen, mit Magnesiumsulfat getrocknet und im Vakuum eingeengt. Es entsteht als ölige Substanz die Verbindung der Formel (VIIIa-1), die ohne weitere Reinigung in die Folgereaktion eingesetzt wird.
Das rohe Sulfochlorid (VIIIa-1) wird in 10 ml Acetonitril aufgenommen und mit 26 % iger wässriger Ammoniaklösung tropfenweise versetzt, bis eine Probe basische Reaktion zeigt. Der Ansatz wird eingeengt und in Methylenchlorid aufgenommen. Unlösliche Salze werden abfiltriert. Das Filtrat wird mit Wasser gewaschen, getrocknet und eingeengt, der Rückstand mit Diethylether verrieben. Man erhält 1.3 g (5.5 mmol) 3-(4,5-Dihydroisoxazol-3-yl)pyridin-2-sulfonamid (IIa-1) vom Reinheitsgehalt (HPLC) 96.1 % in Form farbloser Kristalle, Schmp, 155-156 °C.
Analog können hergestellt werden:

**Tabelle 2a: Verbindungen der Formel (VIIIa)**

| | | | | | | |
|---|---|---|---|---|---|---|
| 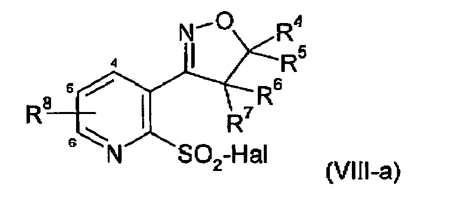 | | | | | | |

| Bsp. Nr. | R⁴ | R⁶ | R⁵ | R⁷ | R⁸ | Hal |
|---|---|---|---|---|---|---|
| VIIIa-1 | H | H | H | H | H | Cl |
| VIIIa-2 | CH(CH₃)₂ | H | H | H | H | Cl |
| VIIIa-3 | CH₃ | H | H | H | H | Cl |
| VIIIa-4 | CH₃ | H | CH₃ | H | H | Cl |
| VIIIa-5 | CH₂CH₂CH₃ | H | H | H | H | Cl |
| VIIIa-6 | CH₂CH₃ | H | H | H | H | Cl |
| VIIIa-7 | CH₃ | CH₃ | H | H | H | Cl |
| VIIIa-8 | H | H | H | H | 6-Cl | Cl |
| VIIIa-9 | H | H | H | H | 6-CH₃ | Cl |
| VIIIa-10 | H | H | H | H | 6-Br | Cl |
| VIIIa-11 | H | H | H | H | 6-F | Cl |
| VIIIa-12 | H | H | H | H | 6-CF₃ | Cl |
| VIIIa-13 | H | H | H | H | 6-OCH₃ | Cl |
| VIIIa-14 | H | H | H | H | 6-SCH₃ | Cl |
| VIIIa-15 | H | H | H | H | 6-SO₂CH₃ | Cl |
| VIIIa-16 | H | H | H | H | 6-I | Cl |
| VIIIa-17 | CH₂CH₂CH₂ | | H | H | H | Cl |
| VIIIa-18 | CH₂CH₂CH₂CH₂ | | H | H | H | Cl |
| VIIIa-19 | CH₂-O-CH₂ | | H | H | H | Cl |
| VIIIa-20 | CH₂-S-CH₂ | | H | H | H | Cl |
| VIIIa-21 | CH₂-O-CH₂CH₂ | | H | H | H | Cl |
| VIIIa-22 | CH₂-S-CH₂CH₂ | | H | H | H | Cl |

**Tabelle 2b: Verbindungen der Formel (VIIIb)**

| | | | | | | |
|---|---|---|---|---|---|---|
| 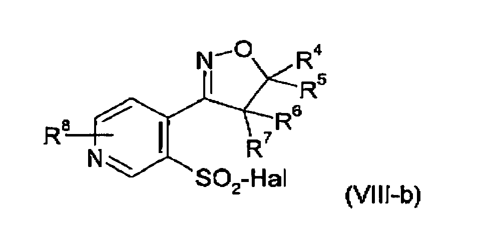 | | | | | | |

| Bsp. Nr. | R⁴ | R⁶ | R⁵ | R⁷ | R⁸ | Hal |
|---|---|---|---|---|---|---|
| VIIIb-1 | H | H | H | H | H | Cl |
| VIIIb-2 | CH(CH₃)₂ | H | H | H | H | Cl |
| VIIIb-3 | CH₃ | H | H | H | H | Cl |
| VIIIb-4 | CH₃ | H | CH₃ | H | H | Cl |
| VIIIb-5 | CH₂CH₂CH₃ | H | H | H | H | Cl |
| VIIIb-6 | CH₂CH₃ | H | H | H | H | Cl |
| VIIIb-7 | CH₃ | CH₃ | H | H | H | Cl |
| VIIIb-8 | CH₂CH₂CH₂ | | H | H | H | Cl |
| VIIIb-9 | CH₂CH₂CH₂CH₂ | | H | H | H | Cl |
| VIIIb-10 | CH₂-O-CH₂ | | H | H | H | Cl |
| VIIIb-11 | CH₂-S-CH₂ | | H | H | H | Cl |
| VIIIb-12 | CH₂-O-CH₂CH₂ | | H | H | H | Cl |
| VIIIb-13 | CH₂-S-CH₂CH₂ | | H | H | H | Cl |

**Tabelle 2c: Verbindungen der Formel (VIIIc)**

| | | | | | | |
|---|---|---|---|---|---|---|
| 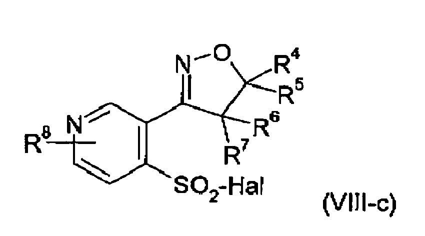 | | | | | | |

| Bsp. Nr. | R⁴ | R⁶ | R⁵ | R⁷ | R⁸ | Hai |
|---|---|---|---|---|---|---|
| VIIIc-1 | H | H | H | H | H | Cl |
| VIIIc-2 | CH(CH₃)₂ | H | H | H | H | Cl |
| VIIIc-3 | CH₃ | H | H | H | H | Cl |
| VIIIc-4 | CH₃ | H | CH₃ | H | H | Cl |
| VIIIc-5 | CH₂CH₂CH₃ | H | H | H | H | Cl |
| VIIIc-6 | CH₂CH₃ | H | H | H | H | Cl |
| VIIIc-7 | CH₃ | CH₃ | H | H | H | Cl |
| VIIIc-8 | CH₂CH₂CH₂ | | H | H | H | Cl |
| VIIIc-90 | CH₂CH₂CH₂CH₂ | | H | H | H | Cl |
| VIIIc-10 | CH₂-O-CH₂ | | H | H | H | Cl |
| VIIIc-11 | CH₂-S-CH₂ | | H | H | H | Cl |
| VIIIc-12 | CH₂-O-CH₂CH₂ | | H | H | H | Cl |
| VIIIc-13 | CH₂-S-CH₂CH₂ | | H | H | H | Cl |

**Tabelle 2d: Verbindungen der Formel (VIIId)**

| | | | | | | |
|---|---|---|---|---|---|---|
| 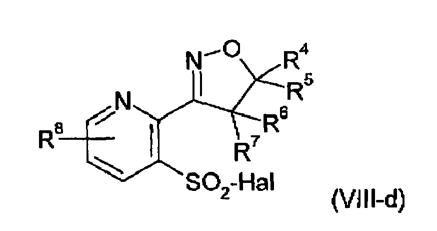 | | | | | | |

| Bsp. Nr. | R⁴ | R⁶ | R⁵ | R⁷ | R⁸ | Hal |
|---|---|---|---|---|---|---|
| VIIId-1 | H | H | H | H | H | Cl |
| VIIId-2 | CH(CH₃)₂ | H | H | H | H | Cl |
| VIIId-3 | CH₃ | H | H | H | H | Cl |
| VIIId-4 | CH₃ | H | CH₃ | H | H | Cl |
| VIIId-5 | CH₂CH₂CH₃ | H | H | H | H | Cl |
| VIIId-6 | CH₂CH₃ | H | H | H | H | Cl |
| VIIId-7 | CH₃ | CH₃ | H | H | H | Cl |
| VIIId-8 | CH₂CH₂CH₂ | | H | H | H | Cl |
| VIIId-9 | CH₂CH₂CH₂CH₂ | | H | H | H | Cl |
| VIIId-10 | CH₂-O-CH₂ | | H | H | H | Cl |
| VIIId-11 | CH₂-S-CH₂ | | H | H | H | Cl |
| VIIId-12 | CH₂-O-CH₂CH₂ | | H | H | H | Cl |
| VIIId-13 | CH₂-S-CH₂CH₂ | | H | H | H | Cl |

**Tabelle 3a: Verbindungen der Formel (IIa)**

| | | | | | | |
|---|---|---|---|---|---|---|
| 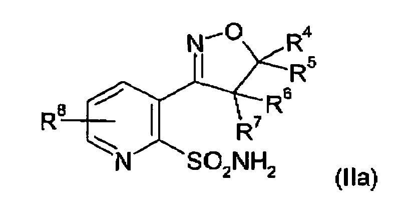 | | | | | | |

| Bsp. Nr. | Phys. Daten Schmp. [°C] | R⁴ | R⁶ | R⁵ | R⁷ | R⁸ |
|---|---|---|---|---|---|---|
| IIa-1 | 155-156 | H | H | H | H | H |
| IIa-2 | 154-155 | CH(CH₃)₂ | H | H | H | H |
| IIa-3 | 152-153 | CH₃ | H | H | H | H |
| IIa-4 | 201-202 | CH₃ | H | CH₃ | H | H |
| IIa-5 | 139-140 | CH₂CH₂CH₃ | H | H | H | H |
| IIa-6 | 173-174 | CH₂CH₃ | H | H | H | H |
| IIa-7 | | CH₃ | CH₃ | H | H | H |
| IIa-8 | | H | H | H | H | 6-Cl |
| IIa-9 | | H | H | H | H | 6-CH₃ |
| IIa-10 | | H | H | H | H | 6-Br |
| IIa-11 | | H | H | H | H | 6-F |
| IIa-12 | | H | H | H | H | 6-CF₃ |
| IIa-13 | | H | H | H | H | 6-OCH₃ |
| IIa-14 | | H | H | H | H | 6-SCH₃ |
| IIa-15 | | H | H | H | H | 6-SO₂CH₃ |
| IIa-16 | | H | H | H | H | 6-I |
| IIa-17 | | CH₂CH₂CH₂ | | H | H | H |
| IIa-18 | | CH₂CH₂CH₂CH₂ | | H | H | H |
| IIa-19 | | CH₂-O-CH₂ | | H | H | H |
| IIa-20 | | CH₂-S-CH₂ | | H | H | H |
| IIa-21 | | CH₂-O-CH₂CH₂ | | H | H | H |
| IIa-22 | | CH₂-S-CH₂CH₂ | | H | H | H |

**Tabelle 3b: Verbindungen der Formel (IIb)**

| | | | | | |
|---|---|---|---|---|---|
| 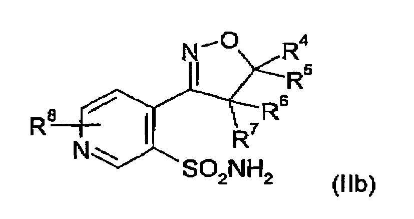 | | | | | |

| Bsp. Nr. | R⁴ | R⁶ | R⁵ | R⁷ | R⁸ |
|---|---|---|---|---|---|
| IIb-1 | H | H | H | H | H |
| IIb-2 | CH(CH₃)₂ | H | H | H | H |
| IIb-3 | CH₃ | H | H | H | H |
| IIb-4 | CH₃ | H | CH₃ | H | H |
| IIb-5 | CH₂CH₂CH₃ | H | H | H | H |
| IIb-6 | CH₂CH₃ | H | H | H | H |
| IIb-7 | CH₃ | CH₃ | H | H | H |
| IIb-8 | CH₂CH₂CH₂ | | H | H | H |
| IIb-9 | CH₂CH₂CH₂CH₂ | | H | H | H |
| IIb-10 | CH₂-O-CH₂ | | H | H | H |
| IIb-11 | CH₂-S-CH₂ | | H | H | H |
| IIb-12 | CH₂-O-CH₂CH₂ | | H | H | H |
| IIb-13 | CH₂-S-CH₂CH₂ | | H | H | H |

**Tabelle 3c: Verbindungen der Formel (IIc)**

| | | | | | | |
|---|---|---|---|---|---|---|
| 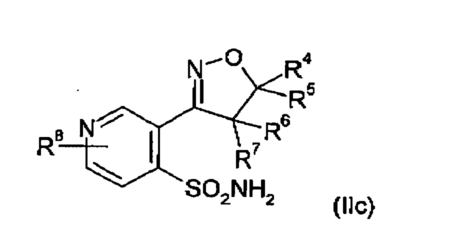 | | | | | | |

| Bsp. Nr. | Phys. Daten | R⁴ | R⁶ | R⁵ | R⁷ | R⁸ |
|---|---|---|---|---|---|---|
| IIc-1 | | H | H | H | H | H |
| IIc-2 | | CH(CH₃)₂ | H | H | H | H |
| IIc-3 | | CH₃ | H | H | H | H |
| IIc-4 | | CH₃ | H | CH₃ | H | H |
| IIc-5 | | CH₂CH₂CH₃ | H | H | H | H |
| IIc-6 | | CH₂CH₃ | H | H | H | H |
| IIc-7 | | CH₃ | CH₃ | H | H | H |
| IIc-8 | | CH₂CH₂CH₂ | | H | H | H |
| IIc-9 | | CH₂CH₂CH₂CH₂ | | H | H | H |
| IIc-10 | | CH₂-O-CH₂ | | H | H | H |
| IIc-11 | | CH₂-S-CN₂ | | H | H | H |
| IIc-12 | | CH₂-O-CH₂CH₂ | | H | H | H |
| IIc-13 | | CH₂-S-CH₂CH₂ | | H | H | H |

**Tabelle 3d: Verbindungen der Formel (IId)**

| | | | | | |
|---|---|---|---|---|---|
| 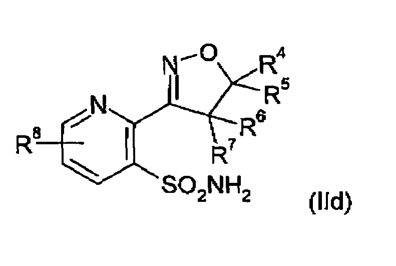 | | | | | |

| Bsp. Nr. | R⁴ | R⁶ | R⁵ | R⁷ | R⁸ |
|---|---|---|---|---|---|
| IId-1 | H | H | H | H | H |
| IId-2 | CH(CH₃)₂ | H | H | H | H |
| IId-3 | CH₃ | H | H | H | H |
| IId-4 | CH₃ | H | CH₃ | H | H |
| IId-5 | CH₂CH₂CH₃ | H | H | H | H |
| IId-6 | CH₂CH₃ | H | H | H | H |
| IId-7 | CH₃ | CH₃ | H | H | H |
| IId-8 | CH₂CH₂CH₂ | | H | H | H |
| IId-9 | CH₂CH₂CH₂CH₂ | | H | H | H |
| IId-10 | CH₂-O-CH₂ | | H | H | H |
| IId-11 | CH₂-S-CH₂ | | H | H | H |
| IId-12 | CH₂-O-CH₂CH₂ | | H | H | H |
| IId-13 | CH₂-S-CH₂CH₂ | | H | H | H |

3. 2-(Benzylhio)-3-(4,5-dihydroisoxazol-3-yl)pyridin (Xa-1) 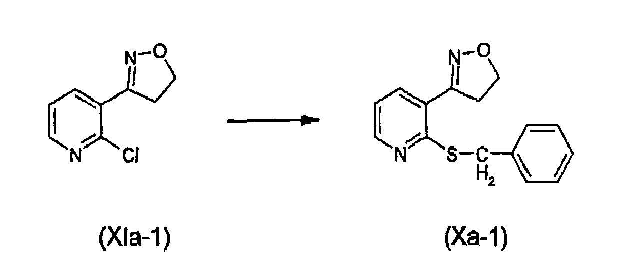 In 600 ml absolutiertem N,N-Dimethylformamid werden 2.86 g (0.11 mol) Natriumhydrid (95 %ig) suspendiert. Unter Rühren werden tropfenweise 13.64 g (0.11 mol) Benzylmercaptan zugefügt, wobei Erwärmung stattfindet.
In die klare Lösung werden portionsweise 18.3 g (0.1 mol) 2-Chloro-3-(4,5-dihydroisoxazol-3-yl)pyridin (Xla-1), gelöst in 30 ml N,N-Dimethylformamid, eingetragen und 3 Stunden bei 20 °C nachgerührt. Die Lösung wird eingeengt, der Rückstand in Methylenchlorid aufgenommen und mehrmals mit Wasser gewaschen. Nach Trocknen mit Magnesiumsulfat, Filtrieren und Einengen wird der Rückstand in Diisopropylether kristallisiert. Man erhält 23.8 g (87.4 mmol) 2-(Benzylthio)-3-(4,5-dihydroisoxazol-3-yl)pyridin (Xa-1) vom Reinheitsgehalt (HPLC) 99.3 % in Form farbloser Kristalle, Schmp. 97-98 °C.
Analog können hergestellt werden:

**Tabelle 4a: Verbindungen der Formel (Xa)**

| | | | | | | |
|---|---|---|---|---|---|---|
| 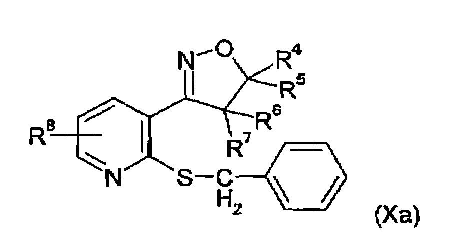 | | | | | | |

| Bsp. Nr. | Phys. Daten Schmp. [°C] | R⁴ | R⁶ | R⁵ | R⁷ | R⁸ |
|---|---|---|---|---|---|---|
| Xa-1 | 97-98 | H | H | H | H | H |
| Xa-2 | 89-90 | CH₃ | H | H | H | H |
| Xa-3 | 53-54 | CH(CH₃)₂ | H | H | H | H |
| Xa-4 | 115-116 | CH₃ | H | CH₃ | H | H |
| Xa-5 | 106-108 | CH₂CH₂CH₃ | H | H | H | H |
| Xa-6 | ¹H-NMR | CH₂CH₃ | H | H | H | H |
| Xa-7 | | CH₃ | CH₃ | H | H | H |
| Xa-8 | | H | H | H | H | 6-Cl |
| Xa-9 | | H | H | H | H | 6-CH₃ |
| Xa-10 | | H | H | H | H | 6-Br |
| Xa-11 | | H | H | H | H | 6-F |
| Xa-12 | | H | H | H | H | 6-CF₃ |
| Xa-13 | | H | H | H | H | 6-OCH₃ |
| Xa-14 | | H | H | H | H | 6-SCH₃ |
| Xa-15 | | H | H | H | H | 6-SO₂CH₃ |
| Xa-16 | | H | H | H | H | 6-I |
| Xa-17 | | CH₂CH₂CH₂ | | H | H | H |
| Xa-18 | | CH₂CH₂CH₂CH₂ | | H | H | H |
| Xa-19 | | CH₂-O-CH₂ | | H | H | H |
| Xa-20 | | CH₂-S-CH₂ | | H | H | H |
| Xa-21 | | CH₂-O-CH₂CH₂ | | H | H | H |
| Xa-22 | | CH₂-S-CH₂CH₂ | | H | H | H |

| | | | | | | |
|---|---|---|---|---|---|---|
| ¹H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) Xa-6: δ = 0.96 (t, 3H); 1.6-1.8 (m, 2H); 3.02 (dd, 1 H); 3.41 (dd, 1 H); 4.45 (s, 2H); 4.62 (m, 1 H); 7.14 (dd, 1 H); 7.15-7.33 (m, 3H); 7.40-7.48 (m, 2H); 7.65 (dd, 1 H); 8.45 (dd, 1 H) ppm | | | | | | |

**Tabelle 4b: Verbindungen der Formel (Xb)**

| | | | | | |
|---|---|---|---|---|---|
| 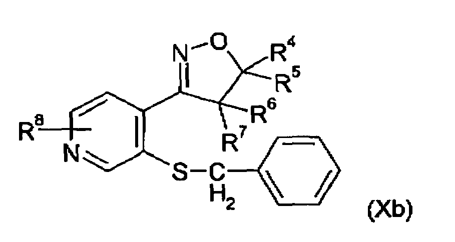 | | | | | |

| Bsp. Nr. | R⁴ | R⁶ | R⁵ | R⁷ | R⁸ |
|---|---|---|---|---|---|
| Xb-1 | H | H | H | H | H |
| Xb-2 | CH₃ | H | H | H | H |
| Xb-3 | CH(CH₃)₂ | H | H | H | H |
| Xb-4 | CH₃ | H | CH₃ | H | H |
| Xb-5 | CH₂CH₂CH₃ | H | H | H | H |
| Xb-6 | CH₂CH₃ | H | H | H | H |
| Xb-7 | CH₃ | CH₃ | H | H | H |
| Xb-8 | CH₂CH₂CH₂ | | H | H | H |
| Xb-9 | CH₂CH₂CH₂CH₂ | | H | H | H |
| Xb-10 | CH₂-O-CH₂ | | H | H | H |
| Xb-11 | CH₂-S-CH₂ | | H | H | H |
| Xb-12 | CH₂-O-CH₂CH₂ | | H | H | H |
| Xb-13 | CH₂-S-CH₂CH₂ | | H | H | H |

**Tabelle 4c: Verbindungen der Formel (Xc)**

| | | | | | |
|---|---|---|---|---|---|
| 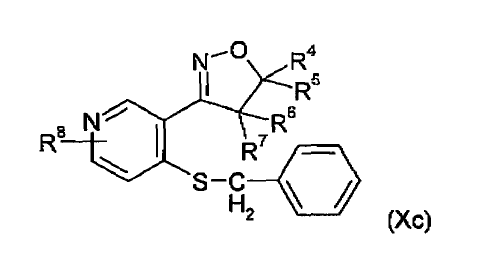 | | | | | |

| Bsp. Nr. | R⁴ | R⁶ | R⁵ | R⁷ | R⁸ |
|---|---|---|---|---|---|
| Xc-1 | H | H | H | H | H |
| Xc-2 | CH₃ | H | H | H | H |
| Xc-3 | CH(CH₃)₂ | H | H | H | H |
| Xc-4 | CH₃ | H | CH₃ | H | H |
| Xc-5 | CH₂CH₂CH₃ | H | H | H | H |
| Xc-6 | CH₂CH₃ | H | H | H | H |
| Xc-7 | CH₃ | CH₃ | H | H | H |
| Xc-8 | CH₂CH₂CH₂ | | H | H | H |
| Xc-9 | CH₂CH₂CH₂CH₂ | | H | H | H |
| Xc-10 | CH₂-O-CH₂ | | H | H | H |
| Xc-11 | CH₂-S-CH₂ | | H | H | H |
| Xc-12 | CH₂-O-CH₂CH₂ | | H | H | H |
| Xc-13 | CH₂-S-CH₂CH₂ | | H | H | H |

**Tabelle 4d: Verbindungen der Formel (Xd)**

| | | | | | |
|---|---|---|---|---|---|
| 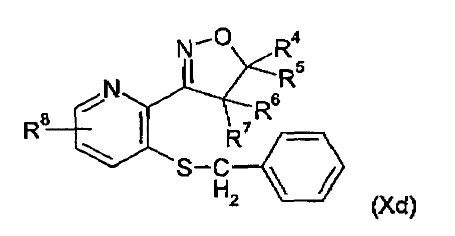 | | | | | |

| Bsp. Nr. | R⁴ | R⁶ | R⁵ | R⁷ | R⁸ |
|---|---|---|---|---|---|
| Xd-1 | H | H | H | H | H |
| Xd-2 | CH₃ | H | H | H | H |
| Xd-3 | CH(CH₃)₂ | H | H | H | H |
| Xd-4 | CH₃ | H | CH₃ | H | H |
| Xd-5 | CH₂CH₂CH₃ | H | H | H | H |
| Xd-6 | CH₂CH₃ | H | H | H | H |
| Xd-7 | CH₃ | CH₃ | H | H | H |
| Xd-8 | CH₂CH₂CH₂ | | H | H | H |
| Xd-9 | CH₂CH₂CH₂CH₂ | | H | H | H |
| Xd-10 | CH₂-O-CH₂ | | H | H | H |
| Xd-11 | CH₂-S-CH₂ | | H | H | H |
| Xd-12 | CH₂-O-CH₂CH₂ | | H | H | H |
| Xd-13 | CH₂-S-CH₂CH₂ | | H | H | H |

4. 2-Chloro-3-(4,5-dihydroisoxazol-3-yl)pyridin (XIa-1) 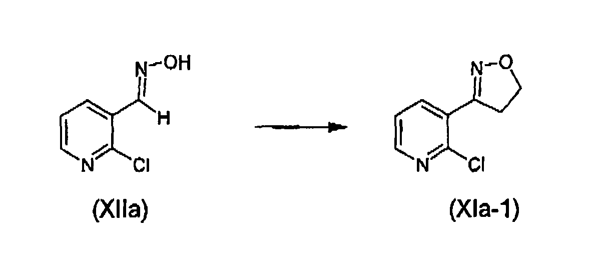 22.5 g (0.14 mol) 2-Chloronicotinaldehyd-oxim werden in einem Druckbehälter in 1650 ml Methylenchlorid gelöst. Der Autoklav wird verschlossen und mit Stickstoff inertisiert. Ethylen wird aufgedrückt, bis 5 bar erreicht sind. Unter gutem Rühren werden 279 g (231.3 ml) Natrium-hypochlorit-Lösung (10-13 % ig) im Verlauf von 1 Stunde bei 20 °C zugepumpt. Nach Dosierende wird noch 15 Stunden bei Raumtemperatur nachgerührt. Nach Entspannen des Druckgefäßes wird überschüssiges Ethylen mit Stickstoff ausgeblasen. Die organische Phase wird abgetrennt, mit Wasser gewaschen und mit Baylith TE 144 getrocknet. Nach Entfernen des Lösungsmittels im Vakuum erhält man 23.8 g 2-Chloro-3-(4,5-dihydroisoxazol-3-yl)pyridin (XIa-1) vom Reinheitsgehalt (HPLC) 98.1 %, logp = 0.97.
Analog können hergestellt werden:

**Tabelle 5a: Verbindungen der Formel (Xla)**

| | | | | | | | |
|---|---|---|---|---|---|---|---|
| 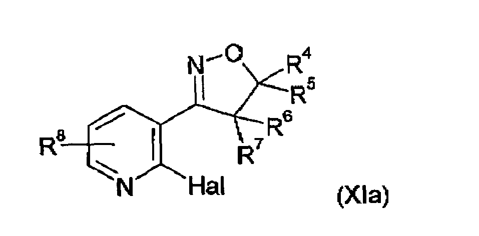 | | | | | | | |

| Bsp. Nr. | Phys. Daten Schmp. [°C] | R⁴ | R⁶ | R⁵ | R⁷ | R⁸ | Hal |
|---|---|---|---|---|---|---|---|
| XIa-1 | 183-185 | H | H | H | H | H | Cl |
| XIa-2 | ¹H-NMR | CH₃ | H | H | H | H | Cl |
| XIa-3 | ¹H-NMR | CH(CH₃)₂ | H | H | H | H | Cl |
| XIa-4 | 48-50 | CH₃ | H | CH₃ | H | H | Cl |
| XIa-5 | 131-133 | CH₂CH₂CH₃ | H | H | H | H | Cl |
| XIa-6 | 132-133 | CH₂CH₃ | H | H | H | H | Cl |
| XIa-7 | 160-161 | CH₃ | CH₃ | H | H | H | Cl |
| XIa-8 | | H | H | H | H | 6-Cl | Cl |
| XIa-9 | | H | H | H | H | 6-CH₃ | Cl |
| XIa-10 | | H | H | H | H | 6-Br | Cl |
| XIa-11 | | H | H | H | H | 8-F | Cl |
| XIa-12 | | H | H | H | H | 6-CF₃ | Cl |
| XIa-13 | | H | H | H | H | 6-OCH₃ | Cl |
| XIa-14 | | H | H | H | H | 6-SCH₃ | Cl |
| XIa-15 | | H | H | H | H | 6-SO₂CH₃ | Cl |
| XIa-16 | | H | H | H | H | 6-I | Cl |
| XIa-17 | | CH₂CH₂CH₂ | | H | H | H | Cl |
| XIa-18 | | CH₂CH₂CH₂CH₂ | | H | H | H | Cl |
| XIa-19 | | CH₂-O-CH₂ | | H | H | H | Cl |
| XIa-20 | | CH₂-S-CH₂ | | H | H | H | Cl |
| XIa-21 | | CH₂-O-CH₂CH₂ | | H | H | H | Cl |
| XIa-22 | | CH₂-S-CH₂CH₂ | | H | H | H | Cl |

| | | | | | | | |
|---|---|---|---|---|---|---|---|
| ¹H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) XIa-2: δ = 1.38 (d, 3H); 3.07 (dd, 1 H); 3.54 (dd, 1 H); 4.88 (m, 1 H); 7.38 (dd, 1 H); 7.96 (dd, 1 H); 8.40 (dd, 1H) ppm XIa-3: δ = 0.95 (d, 3H); 0.99 (d. 3H); 3.19 (dd, 1 H); 3.44 (dd, 1 H); 4.52 (m, 1 H); 7.38 (dd, 1H); 7.94 (dd, 1H); 8.40 (dd, 1 H) ppm | | | | | | | |

**Tabelle 5b: Verbindungen der Formel (XIb)**

| | | | | | | |
|---|---|---|---|---|---|---|
| 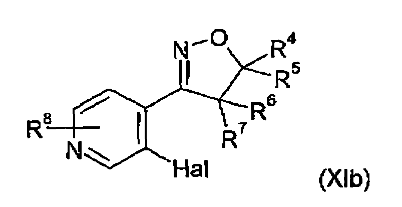 | | | | | | |

| Bsp. Nr. | R⁴ | R⁶ | R⁵ | R⁷ | R⁸ | Hal |
|---|---|---|---|---|---|---|
| XIb-1 | H | H | H | H | H | Cl |
| XIb-2 | CH₃ | H | H | H | H | Cl |
| XIb-3 | CH(CH₃)₂ | H | H | H | H | Cl |
| XIb-4 | CH₃ | H | CH₃ | H | H | Cl |
| XIb-5 | CH₂CH₂CH₃ | H | H | H | H | Cl |
| XIb-6 | CH₂CH₃ | H | H | H | H | Cl |
| XIb-7 | CH₃ | CH₃ | H | H | H | Cl |
| XIb-8 | CH₂CH₂CH₂ | | H | H | H | Cl |
| XIb-9 | CH₂CH₂CH₂CH₂ | | H | H | H | Cl |
| XIb-10 | CH₂-O-CH₂ | | H | H | H | Cl |
| XIb-11 | CH₂-S-CH₂ | | H | H | H | Cl |
| XIb-12 | CH₂-O-CH₂CH₂ | | H | H | H | Cl |
| XIb-13 | CH₂-S-CH₂CH₂ | | H | H | H | Cl |

**Tabelle 5c: Verbindungen der Formel (XIc)**

| | | | | | | |
|---|---|---|---|---|---|---|
| 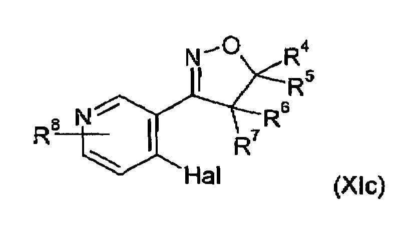 | | | | | | |

| Bsp. Nr. | R⁴ | R⁶ | R⁵ | R⁷ | R⁸ | Hal |
|---|---|---|---|---|---|---|
| XIc-1 | H | H | H | H | H | Cl |
| XIc-2 | CH₃ | H | H | H | H | Cl |
| XIc-3 | CH(CH₃)₂ | H | H | H | H | Cl |
| XIc-4 | CH₃ | H | CH₃ | H | H | Cl |
| XIc-5 | CH₂CH₂CH₃ | H | H | H | H | Cl |
| XIc-6 | CH₂CH₃ | H | H | H | H | Cl |
| XIc-7 | CH₃ | CH₃ | H | H | H | Cl |
| XIc-8 | CH₂CH₂CH₂ | | H | H | H | Cl |
| XIc-9 | CH₂CH₂CH₂CH₂ | | H | H | H | Cl |
| XIc-10 | CH₂-O-CH₂ | | H | H | H | Cl |
| XIc-11 | CH₂-S-CH₂ | | H | H | H | Cl |
| XIc-12 | CH₂-O-CH₂CH₂ | | H | H | H | Cl |
| XIc-13 | CH₂-S-CH₂CH₂ | | H | H | H | Cl |

**Tabelle 5d: Verbindungen der Formel (XId)**

| | | | | | | | |
|---|---|---|---|---|---|---|---|
| 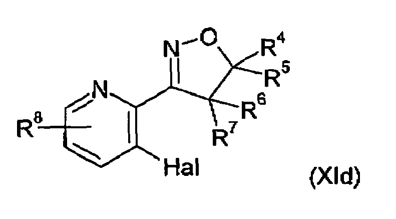 | | | | | | | |

| Bsp. Nr. | Phys. Daten | R⁴ | R⁶ | R⁵ | R⁷ | R⁸ | Hal |
|---|---|---|---|---|---|---|---|
| XId-1 | ¹H-NMR | H | H | H | H | H | Cl |
| XId-2 | | CH₃ | H | H | H | H | Cl |
| XId-3 | | CH(CH₃)₂ | H | H | H | H | Cl |
| XId-4 | | CH₃ | H | CH₃ | H | H | Cl |
| XId-5 | | CH₂CH₂CH₃ | H | H | H | H | Cl |
| XId-6 | | CH₂CH₃ | H | H | H | H | Cl |
| XId-7 | | CH₃ | CH₃ | H | H | H | Cl |
| XId-8 | | CH₂CH₂CH₂ | | H | H | H | Cl |
| XId-9 | | CH₂CH₂CH₂CH₂ | | H | H | H | Cl |
| XId-10 | | CH₂-O-CH₂ | | H | H | H | Cl |
| XId-11 | | CH₂-S-CH₂ | | H | H | H | Cl |
| XId-12 | | CH₂-O-CH₂CH₂ | | H | H | H | Cl |
| XId-13 | | CH₂-S-CH₂CH₂ | | H | H | H | Cl |

| | | | | | | | |
|---|---|---|---|---|---|---|---|
| 1 H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) XId-1: δ = 3.47 (t, 2H); 4.40 (t, 2H); 7.39-7.44 (m, 1 H); 7.58-7.63 (m, 1 H); 8.46 (m, 1 H) ppm | | | | | | | |

### B. Formulierungsbeispiele

a) Ein Stäubemittel wird erhalten, indem man 10 Gew.-Teile einer Verbindung der Formel (I) und 90 Gew.-Teile Talkum als inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z. B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösemittel und 10 Gew.-Teilen oxethyliertes Nonylphenol als Emulgator.
e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | |
|---|---|
| 5 " | 2,2'-dinaphthyimethan-6,6'-disulfonsaures Natrium, |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen werden dann als wässrige Suspension bzw. Emulsion mit einer Wasseraufwandmenge von umgerechnet 600 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Vorauflaufwirksamkeit gegen ein breites Spektrum von Ungräsern und Unkräutern auf. Die erfindungsgemäßen Verbindungen haben beispielsweise eine sehr gute herbizide Wirkung gegen Schadpflanzen wie beispielsweise Alopecurus myosuroides, Matricaria inodora und Stellaria media im Vorauflaufverfahren bei einer Aufwandmenge von 0.08 kg und weniger Aktivsubstanz pro Hektar.

Folgende Ergebnisse wurden mit den erfindungsgemäßen Verbindungen im Vorauflauf erreicht:

| Verbindung | Dosiermenge | Einheit | ALOMY | MATIN | STEME |
|---|---|---|---|---|---|
| Ia-1 | 80 | g/ha | 90 | 90 | 90 |
| Ia-2 | 80 | g/ha | 90 | 90 | 90 |
| Ia-3 | 80 | g/ha | 80 | 80 | 80 |
| Ia-4 | 80 | g/ha | 80 | 90 | 90 |
| Ia-9 | 80 | g/ha | | | 80 |
| Ia-10 | 80 | g/ha | 80 | 80 | 80 |
| Ia-11 | 80 | g/ha | 90 | 80 | 80 |
| Ia-13 | 80 | g/ha | 90 | 80 | 80 |
| Ia-15 | 80 | g/ha | 80 | 80 | |
| Ia-16 | 80 | g/ha | 80 | 80 | |
| Ia-17 | 80 | g/ha | 90 | | |
| Ia-19 | 80 | g/ha | 80 | | 80 |
| Ia-20 | 80 | g/ha | 80 | | |
| Ia-21 | 80 | g/ha | | 80 | 80 |
| Ia-22 | 80 | g/ha | | | 100 |
| Ia-23 | 80 | g/ha | | 80 | |
| Ia-24 | 80 | g/ha | 90 | 90 | 90 |

In der Tabelle weisen die einzelnen Kulturen die folgenden Abkürzungen auf:
- ALOMY:: Acker-Fuchsschwanz(gras) (*Alopecurus myosuroides*)
- MATIN:: Geruchlose Kamille (*Matricaria inodora*)
- STEME:: Vogelmiere (*Stellaria media*)

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen werden dann als wäßrige Suspension bzw. Emulsion mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise haben die erfindungsgemäßen Verbindungen eine sehr gute herbizide Wirkung gegen Schadpflanzen wie beispielweise Alopecurus myosuroides, Echinochloa crus-galli, Lolium multiflorum, Abutilon theophrasti, Amaranthus retroflexus, Pharbitis purpurea, Stellaria media und Viola tricolor im Nachauflaufverfahren bei einer Aufwandmenge von 0.08 kg und weniger Aktivsubstanz pro Hektar.

Folgende Ergebnisse wurden mit den erfindungsgemäßen Verbindungen im Nachauflauf erreicht:

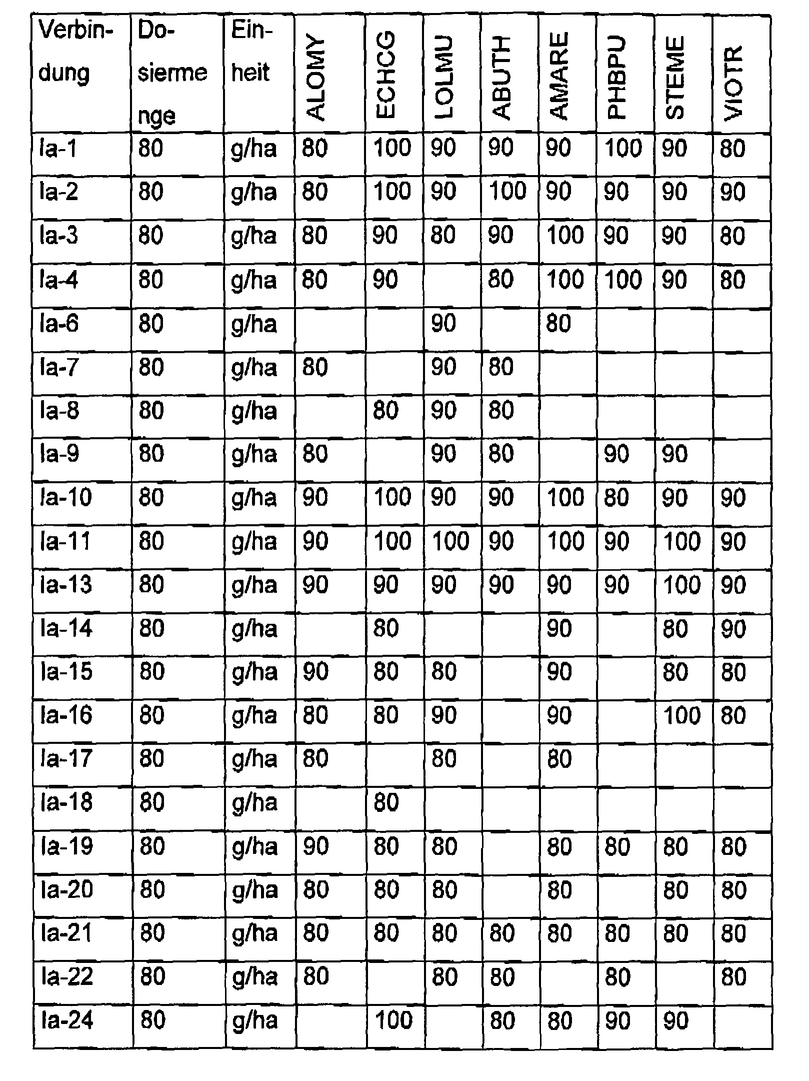

In der Tabelle weisen die einzelnen Kulturen die folgenden Abkürzungen auf:
- ALOMY:: Acker-Fuchsschwanz (*Alopecurus myosuroides*.)
- ECHCG:: Hühnerhirse (*Echinochloa crus-galli*)
- LOLMU:: Vielblütiger Lolch (*Lolium multiflorum*)
- ABUTH:: Samtpappel (*Abutilon theophrasti*)
- AMARE:: Zurückgekrümmter Fuchsschwanz (*Amaranthus retroflexus*)
- PHBPU:: Purpur-Prachtwinde (*Pharbitis* / *Ipomoea purpurea*)
- STEME:: Vogelmiere (*Stellaria media)*
- VIOTR:: Wildes Stiefmütterchen (*Viola tricolor*)

## Patentansprüche

1. N-Azinyl-N'-pyridylsulfonyl-hamstoffe der allgemeinen Formel (I), 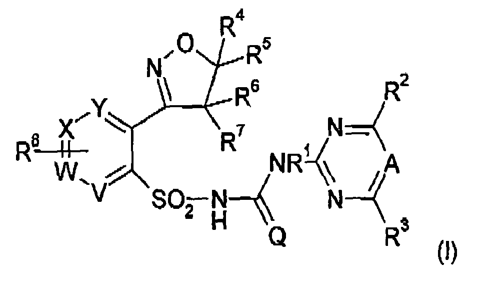 in welcher
V, W, X und Y so gewählt werden, dass eine dieser Indizes für Stickstoff steht und die übrigen Indizes für Kohlenstoffatome stehen, welche unsubstituiert oder aber mit dem dargestellten Rest R⁸ substituiert sein können;
A ausgewählt ist aus der Gruppe, bestehend aus Stickstoff und CR⁹;
wobei
R⁹ ausgewählt ist aus der Gruppe, bestehend aus Wasserstoff, Alkyl, Halogen und Haloalkyl;
R¹ ausgewählt ist aus der Gruppe, bestehend aus Wasserstoff und einem gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl;
R² ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino:
R³ ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino;
R⁴ bis R⁷, jeweils unabhängig voneinander, ausgewählt werden aus der Gruppe, bestehend aus Wasserstoff, Halogen, Cyano, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl oder Dialkylaminocarbonyl, wobei die Reste unsubstituiert sein können oder ein oder mehrere Reste ausgewählt aus der Gruppe, bestehend aus Halogen, Cyano, Alkoxy oder Alkylthio, tragen können, oder R⁴ und R⁶ bzw. R⁵ und R⁷ eine gegebenenfalls durch Sauerstoff oder Schwefel unterbrochene Alkyliden-Gruppe darstellen;
R⁸ ausgewählt ist aus der Gruppe, bestehend aus Wasserstoff, Halogen, Cyano, Thiocyanato, Nitro, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl, oder Dialkylaminocarbonyl, wobei die Reste unsubstituiert sein können oder ein oder mehrere Reste ausgewählt aus der Gruppe, bestehend aus Halogen, Cyano, Alkoxy und Alkylthio, tragen können;
Q ausgewählt ist aus der Gruppe, bestehend aus Sauerstoff, oder Schwefel
sowie Salze von Verbindungen der Formel (I).

2. N-Azinyl-N'-pyridylsulfonyl-hamstoffe nach Anspruch 1, **dadurch gekennzeichnet, dass** der Substituent A ausgewählt ist aus der Gruppe, bestehend aus Stickstoff und CH.

3. N-Azinyl-N'-pyridysulfonyl-hamstoffe nach Anspruch 1 oder 2, **dadurch gekennzeichnet, dass** der Substituent R¹ ausgewählt ist aus der Gruppe, bestehend aus Wasserstoff, gegebenenfalls durch Halogen substituiertes Alkyl, gegebenenfalls durch Halogen substituiertes Alkoxy, gegebenenfalls durch Halogen substituiertes Alkoxyalkyl, gegebenenfalls durch Halogen substituiertes Alkenyl und gegebenenfalls durch Halogen substituiertes Alkinyl.

4. N-Azinyl-N'-pyridylsulfonyl-hamstoffe nach einem der Ansprüche 1 bis 3, **dadurch gekennzeichnet, dass** der Substituent R² ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino und gegebenenfalls durch Halogen substituiertes Dialkylamino.

5. N-Azinyl-N'-pyridysulfonyl-hamstoffe nach einem der Ansprüche 1 bis 5, **dadurch gekennzeichnet, dass** der Substituent R³ ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino und gegebenenfalls durch Halogen substituiertes Dialkylamino.

6. N-Azinyl-N'-pyridysulfonyl-hamstoffe nach einem der Ansprüche 1 bis 5, **dadurch gekennzeichnet, dass** die Substituenten R⁴ bis R⁷, jeweils unabhängig voneinander, ausgewählt sind aus der Gruppe, bestehend aus Wasserstoff, Halogen, Cyano, Thiocyanato, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylsulfinyl, gegebenenfalls durch Halogen substituiertes Alkylsulfonyl, gegebenenfalls durch Halogen substituiertes Alkylamino, gegebenenfalls durch Halogen substituiertes Alkylcarbonyl, gegebenenfalls durch Halogen substituiertes Alkoxycarbonyl oder gegebenenfalls durch Halogen substituiertes Alkylaminocarbonyl.

7. N-Azinyl-N'-pyridysulfonyl-hamstoffe nach einem der Ansprüche 1 bis 6, **dadurch gekennzeichnet, dass** der Substituent R⁸ ausgewählt ist aus der Gruppe, bestehend aus Wasserstoff, Halogen, Cyano, Thiocyanato, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylsulfinyl, gegebenenfalls durch Halogen substituiertes Alkylsulfonyl, gegebenenfalls durch Halogen substituiertes Alkylamino, gegebenenfalls durch Halogen substituiertes Alkylcarbonyl, gegebenenfalls durch Halogen substituiertes Alkoxycarbonyl und gegebenenfalls durch Halogen substituiertes Alkylaminocarbonyl.

8. Verfahren zur Herstellung von N-Azinyl-N'-pyridylsulfonyl-hamstoffen gemäß einem der Ansprüche 1 bis 7, **gekennzeichnet durch** einen der folgenden Verfahrensschritte:
(a) Umsetzung von (4,5-Dihydroisoxazol-3-yl)pyridin-sulfonamide der allgemeinen Formel (II) 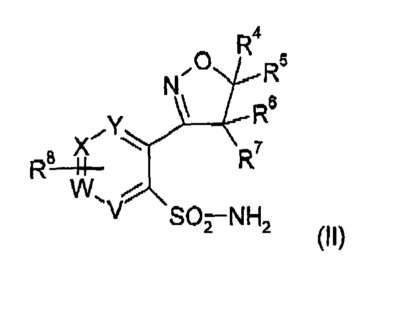 mit einem heterocyclischen (Thio)-Carbamat der allgemeinen Formel (I-II) 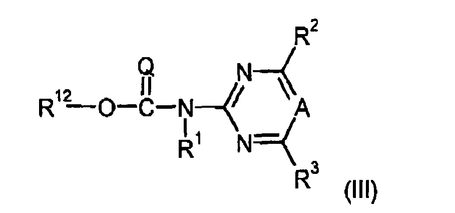 worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet und worin V, W, X, Y, R¹ bis R⁸, Q und A die Bedeutung gemäß einem der Ansprüche 1 bis 7 aufweisen; oder
(b) Umsetzung von (4,5-Dihydroisoxazol-3-yl)pyridin-sulfonsäureiso(thio)-cyanate der allgemeinen Formel (IV) 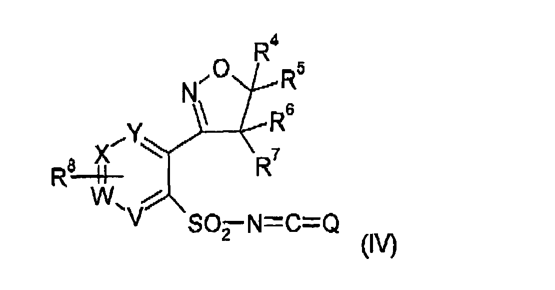 mit einem Aminoheterocyclus der allgemeinen Formel (V) 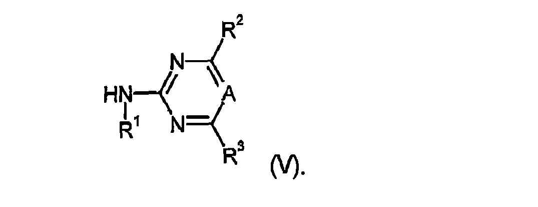 worin V, W, X, Y, R¹ bis R⁸, Q und A die Bedeutung gemäß einem der Ansprüche 1 bis 7 aufweisen; oder
(c) Umsetzung von (4,5-Dihydroisoxazol-3-yl)pyridin-sulfonyl(thio) carbamate der allgemeinen Formel (VI) 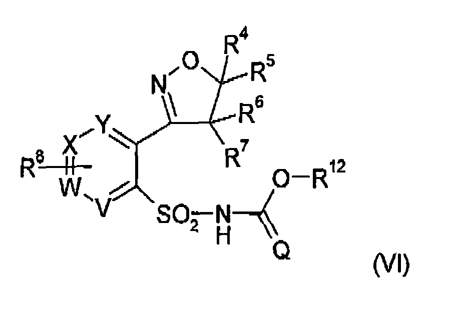 worin R¹² ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet, mit einem Aminoheterocyclus der allgemeinen Formel (V) 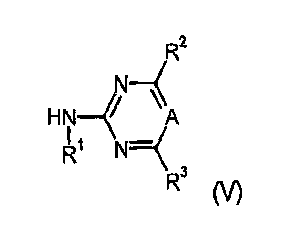 umsetzt, worin V, W, X, Y, R¹ bis R⁸, Q und A die Bedeutung gemäß einem der Ansprüche 1 bis 7 aufweisen; oder
(d) Umsetzung von (4,5-Dihydroisoxazol-3-yl)pyridin-sulfonamide der allgemeinen Formel (II) 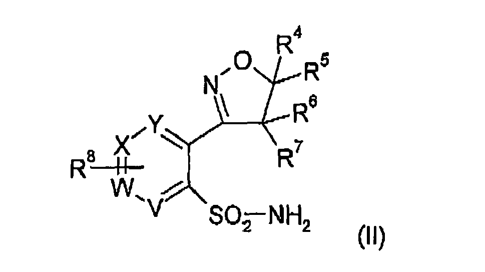 mit einem Iso(thio)cyanat der allgemeinen Formel (VII) 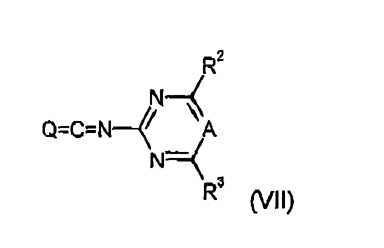 gegebenenfalls in Gegenwart eines Reaktionshilfsmittels,, worin R¹ Wasserstoff bedeutet und V, W, X, Y, R² bis R⁸, Q und A die Bedeutung gemäß einem der Ansprüche 1 bis 7 aufweisen; oder
(e) Umsetzung von einem Aminoheterocyclus der allgemeinen Formel (V) 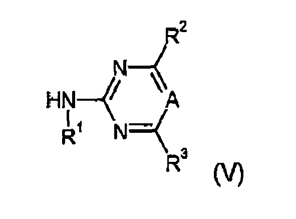 zunächst basenkatalysiert mit einem Kohlensäureester und das gebildete Intermediat der allgemeinen Formel (III) 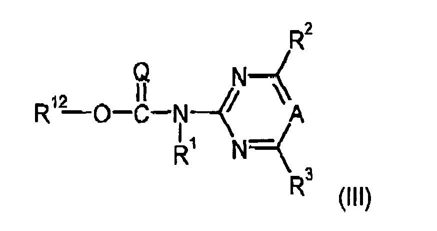 in einer Eintopfreaktion mit einem (4,5-Dihydroisoxazol-3-yl)pyridin-sulfonamid der allgemeinen Formel (II) 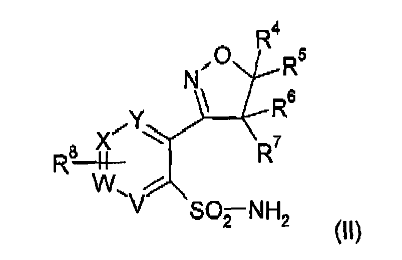 umsetzt, worin V, W, X, Y, R¹ bis R⁸, Q und A die Bedeutung gemäß einem der Ansprüche 1 bis 7 aufweisen; oder
(f) Umsetzung von (4,5-Dihydroisxazol-3-yl)pyridin-sulfonsäurehalogenide der allgemeinen Formel (VIII) 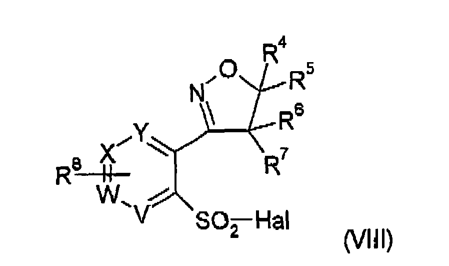 wobei Hal ein Halogenatom ist mit einem (Thio)-Cyanat zu einem I-so(thio)cyanat der allgemeinen Formel (IV) 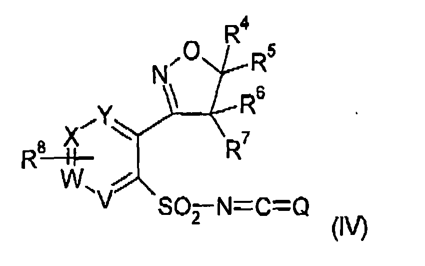 oder einem solvatisierten (stabilisierten) Derivat, und anschließend mit einem Aminoheterocyclus der allgemeinen Formel (V) 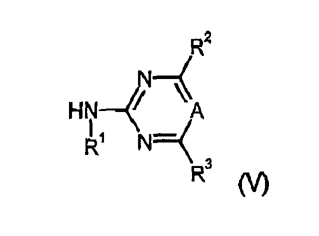 umsetzt, worin V, W, X, Y, R¹ bis R⁸, Q und A die Bedeutung gemäß einem der Ansprüche 1 bis 7 aufweisen; oder
(g) Umsetzung von (4,5-Dihydroisoxazol-3-yl)pyridin-sulfonsäureamide der allgemeinen Formel (II) mit einem heterocyclischen Bis-carbamat der allgemeinen Formel (IX), 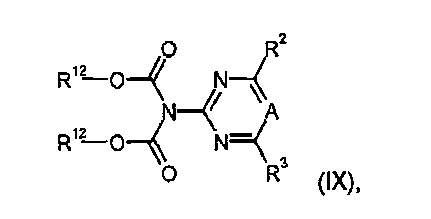 worin R¹² ein substituierter oder unsubstituierter (C₁-C₂₀)-Kohlenwasserstoffrest bedeutet, in Gegenwart eines basischen Reaktionshilfsmittels, worin Q Sauerstoff bedeutet und V, W, X, Y, R¹ bis R⁸, und A die Bedeutung gemäß einem der Ansprüche 1 bis 7 aufweisen; oder
(h) Umsetzung von (4,5-Dihydroisoxazol-3-yl)pyridin-sulfonsäureamide der allgemeinen Formel (II) 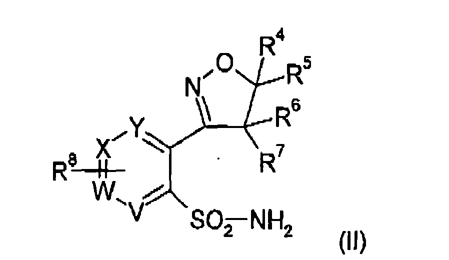 basenkatalysiert mit einem Kohlensäureester und Umsetzung des gebildeten Intermediats der allgemeinen Formel (VI) 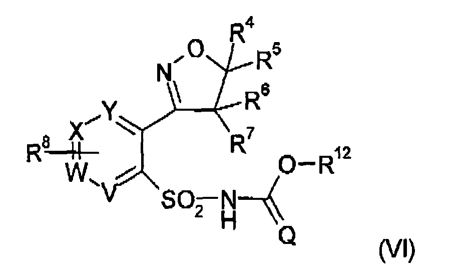 in einer Eintopfreaktion mit einem Aminoheterocyclus der allgemeinen Formel (V) 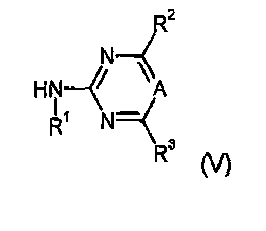 umsetzt, worin V, W, X, Y, R¹ bis R⁸, Q und A die Bedeutung gemäß einem der Ansprüche 1 bis 7 aufweisen.

9. Verbindungen der allgemeinen Formel (II) 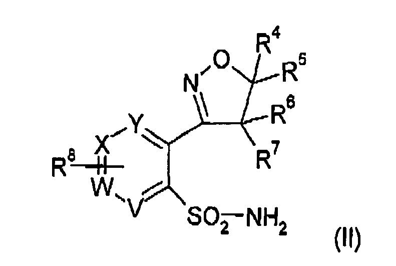 in welchen die Reste V, W, X, Y, R⁴, R⁵, R⁶, R⁷ und R⁸ die Bedeutungen gemäß einem der Ansprüche 1 bis 7 aufweisen.

10. Verbindungen der allgemeinen Formel (VIII) 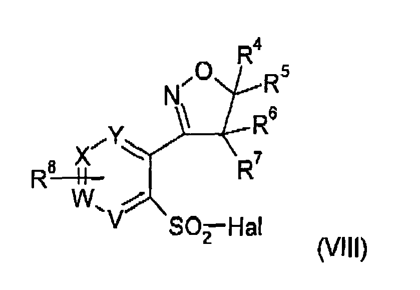 in welchen die Reste V, W, X, Y, R⁴, R⁵, R⁶, R⁷, R⁸ und Hal die Bedeutungen gemäß einem der Ansprüche 1 bis 7 aufweisen.

11. Verbindungen der allgemeinen Formel (XI) 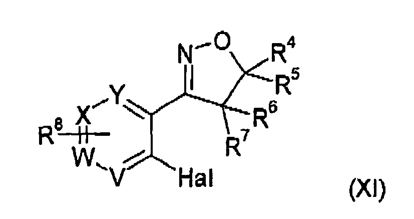 in welchen die Reste V, W, X, Y, R⁴, R⁵, R⁶, R⁷, R⁸ und Hal die Bedeutungen gemäß einem der Ansprüche 1 bis 7 aufweisen.

12. Verbindungen der allgemeinen Formel (X) 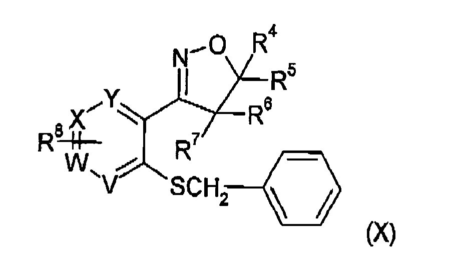 in welchen die Reste V, W, X, Y, R⁴, R⁵, R⁶, R⁷ und R⁸ die Bedeutungen gemäß einem der Ansprüche 1 bis 7 aufweisen.

13. Zusammensetzungen, enthaltend mindestens eine Verbindung der allgemeinen Formel (I) gemäß einem der Ansprüche 1 bis 7.

14. Zusammensetzung nach Anspruch 13, **dadurch gekennzeichnet, dass** die Zusammensetzung mindestens einen weiteren Wirkstoff umfasst, welcher ausgewählt ist aus der Gruppe, bestehend aus mindestens einem weiteren Herbizid und mindestens einem Safener.

15. Verwendung der Verbindungen der allgemeinen Formel (I) gemäß einem der Ansprüche 1 bis 7 als Herbizide und Pflanzenwachstumsregulatoren.

16. Verwendung der Zusammensetzungen gemäß einem Anspruch 14 oder 15 als Herbizide und Pflanzenwachstumsregulatoren.

17. Verwendung nach Anspruch 15 oder 16 zur Pflanzenbekämpfung in speziellen Pflanzenkulturen oder als Pflanzenschutzregulator.
